# EUROPÄISCHE PATENTSCHRIFT

(11) **EP 4 098 233 B1**
(45) Veröffentlichungstag und Bekanntmachung des Hinweises auf die Patenterteilung: **06.08.2025**
(21) Anmeldenummer: 22180243.2
(22) Anmeldetag: 08.03.2017
(51) Int. Cl.: A61F 9/008

(54) **OPHTHALMOLOGISCHES LASERTHERAPIESYSTEM**
OPTHALMOLOGICAL LASER THERAPY SYSTEM
SYSTÈME DE TRAITMENT OPHTHALMOLOGIQUE AU LASER

(30) Priorität: 11.03.2016 DE 102016204032
(43) Veröffentlichungstag der Anmeldung: 07.12.2022
(62) Teilanmeldung aus: 17709648.4
(73) Patentinhaber: Carl Zeiss Meditec AG, 07745 Jena (DE)
(72) Erfinder: Festag, Karsten, 07745 Jena (DE)
(74) Vertreter: Rößner, Ulrike

(56) Entgegenhaltungen:
- US-A1- 2010 042 081
- US-A1- 2014 107 634

## Beschreibung

Die vorliegende Erfindung betrifft ein ophthalmologisches Lasertherapiesystem mit einer Gerätebasis und einem Gerätekopf, die zueinander mittels einer Translationsbewegung verfahrbar sind, und mit einer Lasereinrichtung, die eine Laserquelle und eine erste Lasertherapieoptik und einen Laserschwenkarm mit einer zweiten Lasertherapieoptik und einer Laseraustrittsöffnung enthält.

In der ophthalmologischen Lasertherapie ist es mittlerweile üblich, eine Lasertherapievorrichtung zur Erzeugung von Schnitten in einem Augengewebe, oder zur Ablation oder zur Koagulation eines Augengewebes mittels Laserstrahlung zu kombinieren mit einer Untersuchungsvorrichtung - entweder durch Integration in ein gemeinsames System oder durch Installation der Untersuchungsvorrichtung in örtlicher Nähe der Lasertherapievorrichtung, um einen Wechsel zwischen den beiden Vorrichtungen zu ermöglichen, ohne den Patienten in einen anderen Raum zu verbringen.

Dies ist beispielsweise sehr vorteilhaft in der laserunterstützten Augenchirurgie zur Korrektur von Fehlsichtigkeit oder zur Therapie von anderen Augenerkrankungen wie z.B. einem Katarakt mittels der Kataraktchirurgie, wo sich Arbeitsschritte zur Charakterisierung der Augenstrukturen mit chirurgischen Schritten und Schritten zur Verifikation des chirurgischen Eingriffs oder zur Unterstützung des chirurgischen Eingriffs abwechseln. So können die Augenstrukturen zunächst mittels der optischen Kohärenztomographie (OCT) oder mittels Ultraschall charakterisiert werden. Hernach kann mittels eines gepulsten Laserstrahls ein Augengewebe geschnitten, in diesem Falle also mittels Photodisruption getrennt werden. Das Resultat kann dann mittels eines Operationsmikroskops verifiziert werden und Folgeschritte, wie beispielsweise das Absaugen einer zuvor mit dem Laserstrahl zerschnittenen und/oder durch Ultraschall zertrümmerten getrübten Augenlinse in der Kataraktchirurgie, unter Kontrolle durch das Operationsmikroskop durchgeführt werden.

Auch bei Fehlsichtigkeitskorrekturen wie beispielsweise zur Durchführung einer "SMILE"-Behandlung, also einer Lentikelextraktion durch kleine Inzision ("Small Incision Lenticule Extraction"), sind nach der Vorbereitung des Patienten und entsprechender Charakterisierung der Augenstrukturen zwei Hauptarbeitsschritte durchzuführen: Zum einen die Lasertherapie, bei der das Patientenauge mittels eines Kontaktglases an die Laseroptik kontaktiert wird und Laserschnitte am Auge ausgeführt werden, und zum anderen die Lentikelextraktion, bei der vom Operateur unter Beobachtung durch ein Operationsmikroskop das während der Lasertherapie geschnittene Lentikel entfernt wird.

Ähnliches gilt für Implantationen von Lentikeln in ein Patientenauge: Hier wird zunächst laserunterstützt durch Trennung von Augengewebe ein entsprechender Aufnahmebereich in diesem Augengewebe geschaffen, in der Regel in dem Hornhautgewebe des Auges, und anschließend unter Beobachtung beispielsweise durch ein Operationsmikroskop ein Implantat in den Aufnahmebereich eingeführt und angepasst.

Mit den derzeit üblichen Verfahren werden diese Arbeitsschritte an räumlich getrennten Positionen durchgeführt. Beispielsweise befindet sich bei der "SMILE"-Behandlung mittels des "VisuMax" die Lasertherapie-Position ca. 200 mm hinter und ca. 100 mm oberhalb der Lentikelextraktions-Position.

Unter Zuhilfenahme von ophthalmologischen Lasertherapiesystemen nach dem Stand der Technik muss also die Relativposition zwischen der jeweiligen Behandlungsvorrichtung und dem Patienten zwischen den verschiedenen Arbeitsschritten signifikant verändert werden. Diese Umpositionierung über einen großen Weg führt zu drei wesentlichen Nachteilen:
Sie erfordert einen hohen technischen Aufwand zur Realisierung eines großen Bewegungsbereiches. Sie erfordert zudem einen hohen Platzbedarf - sowohl für den Bauraum des ophthalmologischen Lasertherapiesystems als auch als freizuhaltender Bereich für die Bewegung. Die Verfahrensdauer ist relativ hoch, weil zwischen den Arbeitsschritten eine längere Zeit zum Umpositionieren erforderlich ist, denn die Verfahrgeschwindigkeit beim Umpositionieren ist aus Sicherheitsgründen klein.

Die erforderliche Beweglichkeit zum Umpositionieren des Patienten bzw. des Patientenauges kann nach dem Stand der Technik in der Patientenlagerungseinrichtung vorgesehen sein. Ebenso kann sie in den erforderlichen Systemen, also der Lasereinrichtung und den Untersuchungseinrichtungen, vorhanden sein: Insbesondere bei nichtintegrierten Systemen ist hierfür der Platzbedarf enorm.

Ein weiterer großer Nachteil bei den bekannten Geräten ist die Einschränkung des Freiraumes für den Ein- und Ausstieg des Patienten durch die in der Regel über der Patientenlagerungseinrichtung befindliche Laseroptik. Hierzu wird in der Druckschrift WO 2012/152496 A1 eine Methode veröffentlicht, bei der die Lasertherapieoptik um 90° nach oben geschwenkt wird und im Gehäuse des Grundgerätes aufgenommen wird. Diese Ausführung hat jedoch den Nachteil, dass die Lasertherapieoptik in ihrer Arbeitsposition relativ ungeschützt vor äußeren mechanischen Einwirkungen ist.

Die Druckschrift US 2010/042081 A1 zeigt ein Lasertherapiegerät mit einem Gerätekopf, der auf einer Gerätebasis lateral beweglich ist, und an seinem Ende einen um eine Achse begrenzt schwenkbare Laseraustrittsöffnung aufweist, was die zur Vor- und Nachbereitung benötigte Arbeitsfreiheit erhöht. An diesem Lasertherapiegerät bleibt jedoch die Lasertherapieoptik trotz der lateralen Beweglichkeit des Gerätekopfes in jeder Stellung exponiert und ungeschützt.

Die Druckschrift US 2014/107634 A1 beschreibt ein Lasertherapiegerät mit einem an einem Arm befindlichen Operationsmikroskop und einem lateral beweglichen Lasertherapiearm. Beide Arme sind leicht um zueinander parallele Achsen schwenkbar mit dem Ziel, Operationsmikroskop und Lasertherapiearm so übereinander zu positionieren, dass die Lasertherapie mit dem Operationsmikroskop beobachtbar ist. Ein Umpositionieren des Patienten kann damit zwar vermieden werden, aber beide Arme befinden sich ungeschützt im Raum, auch wenn sie nicht benötigt werden. Zudem ist der Raumbedarf für das Gerät nach wie vor groß.

Aufgabe der vorliegenden Erfindung ist es deshalb, ein ophthalmologisches Lasertherapiesystem zu beschreiben, das erlaubt, auf kleinstem Raum und ohne Umpositionieren des Patienten zu arbeiten, die empfindliche Lasertherapieoptik, insbesondere eine schwenkbare Lasertherapieoptik, jedoch trotzdem wirkungsvoll zu schützen.

Die Aufgabe wird gelöst durch ein ophthalmologisches Lasertherapiesystem, das eingerichtet ist, sämtliche Arbeitsschritte einer Lasertherapie am Auge eines Patienten so auszuführen, dass der Wirkungsort aller Arbeitsschritte bei maximaler Arbeitsfreiheit eines Operateurs fest bleibt, die Position des Patientenauges während der gesamten Lasertherapie also nicht verändert werden muss. Die Position des Patientenauges im ersten Arbeitsschritt bestimmt die Position des Patientenauges aller nachfolgenden Arbeitsschritte und damit den Wirkungsort ihrer jeweils notwendigen Einrichtungen.

Unter einer ophthalmologischen Lasertherapie soll dabei jegliche Therapie verstanden werden, bei der ein Laserstrahl so in ein Gewebe eines Auges fokussiert wird, dass er das Gewebe verändert. Die ophthalmologische Lasertherapie beinhaltet insbesondere entsprechende chirurgische Eingriffe, bei denen mittels eines Lasers, bevorzugt eines gepulsten Lasers wie beispielsweise eines Femtosekunden-Lasers, durch Photodisruption ein Gewebe des Auges "geschnitten" wird, durch eine Ablationswirkung ein Bereich eines Augengewebes abgetragen oder durch eine Koagulationswirkung Augengewebe miteinander "verklebt" wird bzw. der Brechungsindex des Materials, also eines Augengewebes oder aber eines Implantats, durch die Laserstrahlung verändert wird.

Diese Aufgabe wird insbesondere gelöst durch ein ophthalmologisches Lasertherapiesystem, das eine Gerätebasis, einen Gerätekopf, eine Lasereinrichtung und einen Laserschwenkarm umfasst.

Während die Gerätebasis die während eines Lasertherapieverfahrens - inklusive der vor- und nachbereitenden Arbeitsschritte - unbewegliche Basis des ophthalmologischen Lasertherapiesystems darstellt, dient der Gerätekopf insbesondere der Befestigung beweglicher Aufbauten. Er ist zudem wiederum die Grundlage für die gemeinsame Verschiebung dieser Aufbauten im Raum. Hierzu ist der Gerätekopf auf der Gerätebasis mittels einer Translationsbewegung mindestens in einer x-y-Ebene verfahrbar. Die x-y-Ebene ist dabei eine Ebene, die parallel zu einer Standebene bzw. Bodenebene des ophthalmologischen Lasertherapiesystems verläuft. Dabei ist das ophthalmologische Lasertherapiesystem bevorzugt so eingerichtet, dass eine Translationsbewegung in der x-y-Ebene in einer beliebigen Richtung ausgeführt werden kann.

Das ophthalmologische Lasertherapiesystem als Ganzes kann dabei auf dieser Standebene fest installiert sein, oder aber eine Transportmöglichkeit wie beispielsweise ein Rollsystem umfassen, die es gestattet, das System vor oder nach einem entsprechenden Lasertherapieverfahren in eine Parkposition zu bringen.

Die Lasereinrichtung enthält eine Laserquelle und eine erste Lasertherapieoptik, die vorzugsweise im Gerätekopf angeordnet sind. Die Stromversorgung der Lasereinrichtung wie auch die Elektronik ist vorzugsweise in der Gerätebasis angeordnet. Die Laserquelle ist vorzugsweise eingerichtet zur Erzeugung einer gepulsten Laserstrahlung. Insbesondere ist hier eine Femto-Sekunden-Laserquelle einsetzbar. Eine solcher Femto-Sekunden-Laserquelle erzeugt hierfür bevorzugt Pulse einer Pulsdauer von 100fs bis 600 fs mit einer Pulswiederholrate zwischen 100kHz und 100MHz, vorzugsweise 500kHz bis 5MHz, und einer Pulsenergie von 20nJ bis 20µJ, vorzugsweise 100nJ bis 14µJ.

Durch die erste Lasertherapieoptik wird der Laserstrahl in einen Laserschwenkarm mit einer zweiten Lasertherapieoptik und einer Laseraustrittsöffnung übergeben. Die Laseraustrittsöffnung ist dabei der Ort, aus der ein Laserstrahl, insbesondere ein Therapielaserstrahl, aus dem ophthalmologischen Lasertherapiesystem austritt, um anschließend seinen Wirkungsort, beispielsweise in einem Gewebe des Auges, zu erreichen. Dabei kann das Auge eines Patienten mittels eines Patienteninterfaces oder eines Kontaktglases an der Laseraustrittsöffnung fixiert werden, um eine definierte räumliche Beziehung des zu bearbeitenden Gewebes zur Laseraustrittsöffnung herzustellen.

Unter den Begriff Lasertherapieoptik, die sich aus einer ersten Lasertherapieoptik, die in der Regel im Gerätekopf untergebracht ist, und einer zweiten Lasertherapieoptik im Laserschwenkarm zusammensetzt, sollen hier neben optischen Abbildungselementen wie festen und/oder beweglichen Linsen bzw. Linsensystemen auch Strahlführungsmittel fallen. Diese Strahlführungsmittel können feste und/oder bewegliche Strahlführungsmittel sein. Bewegliche Strahlführungsmittel beinhalten hier auch entsprechende Scansysteme. Üblich sind Galvanometerscanner, die mittels drehbarer Spiegel den Laserstrahl in einer Ebene senkrecht zur Strahlachse positionieren können. Es kann aber auch mittels eines Linearantriebs eine Linse quer zur optischen Achse verschoben werden. Zur Veränderung der Tiefenablage des Fokus werden üblicherweise ein oder mehrere Linsen mittels eines Linearantriebs in Strahlrichtung bewegt. Linearantriebe hierfür können z.B. ein Servomotor mit einer Spindel, ein Piezo-Antrieb oder ein Moving-Coil bzw. Moving-Magnet-Antrieb sein.

Die Lasertherapieoptik dient also der Lenkung und Formung des Laserstrahls von seinem Austritt aus der Laserquelle bis hin zu seinem Wirkungsort, in der Regel dem Gewebe eines Auges. Insbesondere dient sie zudem der entsprechenden gewünschten Verschiebung des Laserstrahls nach einem geplanten Scanmuster während eines Lasertherapieschritts.

Prinzipiell ist hier beispielsweise ein optisches Konzept wie in der WO 2006/102971 A2 beschrieben einsetzbar.

Der Laserschwenkarm ist dabei am Gerätekopf um eine erste Achse schwenkbar befestigt. Diese erste Achse ist bevorzugt eine horizontale Achse, also eine Achse, die parallel zur x-y-Ebene verläuft.

Der Laserschwenkarm ist insbesondere zwischen einer Ruheposition und einer Arbeitsposition schwenkbar, wobei die Ruheposition eine Position darstellt, in der der Laserschwenkarm "verstaut" ist, um in Arbeitsschritten, in denen kein Laserstrahl benötigt wird, maximale Arbeitsfreiheit für den Operateur oder aber für weitere Einrichtungen zu gewähren. In der Regel ist also die Ruheposition eine Position, in der der Laserschwenkarm senkrecht nach oben geschwenkt ist. Die Arbeitsposition hingegen ist eine Position, in der der Laserschwenkarm in eine vorzugsweise waagerechte Position über den Patienten verbracht ist.

Dabei werden in einer Ausgestaltung dieses ophthalmologischen Lasertherapiesystems neben der waagerechten Arbeitsposition verschiedene weitere Arbeitspositionen des Laserschwenkarms ermöglicht: Hierzu ist in dieser Ausgestaltung die erste und zweite Lasertherapieoptik entsprechend eingerichtet. Sie enthält hierfür Mittel bzw. eine Anordnung von optischen Elementen bzw. Scanelementen derart, dass die Führung des Laserstrahls durch den Laserschwenkarm anpassbar ist an die gewünschte Arbeitsposition des Laserschwenkarms. Dies kann beispielsweise durch schwenkbare Spiegel anstelle von festen Spiegeln sowohl beim Eintritt in die zweite Lasertherapieoptik, also die Optik des Laserschwenkarms, als auch vor der Laseraustrittsöffnung realisiert werden.

Arbeitsposition und Ruheposition können als zwei Endstellungen, ggf. eindeutig identifizierbar mit einem Anschlag, ausgebildet sein.

Der Laserschwenkarm ist damit in der Regel ein um eine Achse am Gerätekopf schwenkbarer Arm, der ansonsten starr ausgeführt ist, also keine weiteren Schwenkachsen aufweist. Durch die Kombination der Schwenkbewegung des Laserschwenkarms um diese erste Achse mit der Transversalbewegung in einer x-y-Ebene ist eine Bewegung im Raum möglich.

Des Weiteren sorgt eine Steuereinheit für die Steuerung des ophthalmologischen Lasertherapiesystems, insbesondere der Translationsbewegung des Gerätekopfes auf der Gerätebasis und der Schwenkbewegung des Laserschwenkarms, sowie der Lasereinrichtung. Zur Steuerung der Lasereinrichtung gehört insbesondere die Steuerung der Erzeugung eines Laserstrahls, die entsprechende Lenkung des Laserstrahls durch bewegliche Strahlführungsmittel und damit eine Steuerung einer Scanbewegung des Fokus des Laserstrahls. Dabei kann die Steuereinheit auch mehrere örtlich voneinander getrennte Steuereinrichtungen umfassen.

In einer grundlegenden Ausführungsform ist das erfindungsgemäße ophthalmologische Lasertherapiesystem dadurch gekennzeichnet, dass am Gerätekopf weiterhin ein Untersuchungsschwenkarm mit einer Untersuchungseinrichtung um eine zweite Achse schwenkbar befestigt ist. Die Untersuchungseinrichtung des Untersuchungsschwenkarms kann dabei auch eine weitere Therapiemöglichkeit umfassen.

Auch der Untersuchungsschwenkarm ist insbesondere schwenkbar zwischen einer Ruheposition und einer Arbeitsposition. In dieser Ausführungsform weisen sowohl der Laserschwenkarm als auch der Untersuchungsschwenkarm nur eine Arbeitsposition auf. Durch die Untersuchungseinrichtung und ihre Arbeitsposition wird ein Untersuchungsvolumen definiert. Der Laserschwenkarm wiederum bestimmt, insbesondere durch die Position seiner Laseraustrittsöffnung in Arbeitsposition, ein Arbeitsvolumen des Laserstrahls. Dieses Arbeitsvolumen beschreibt ein Volumen, in dem der aus der Laseraustrittsöffnung der Lasereinrichtung austretende Laserstrahl fokussierbar ist und an seinem Fokuspunkt eine Wirkung erzielt - beispielsweise eine Trennwirkung bzw. Schneidwirkung in einem Augengewebe durch Photodisruption. Es ist damit auch das Volumen, in dem der Fokuspunkt des Laserstrahls ohne Abbildungsfehler bzw. unter Korrektur der Abbildungsfehler bewegt werden kann.

Beide Achsen, also die erste Achse des Laserschwenkarms und die zweite Achse des Untersuchungsschwenkarms, sind in ihrer Lage so zueinander angeordnet, dass ein Arbeitsvolumen des Laserstrahls, wenn sich der Laserschwenkarm in einer Arbeitsposition befindet, ein Teilvolumen des Untersuchungsvolumens der Untersuchungseinrichtung am Untersuchungsschwenkarm ist, wenn sich dieser in einer Arbeitsposition befindet. Dabei kann das Teilvolumen auch ein unechtes Teilvolumen sein, d.h. Arbeitsvolumen und Untersuchungsvolumen sind identisch.

In ihren jeweiligen Ruhepositionen sind der Laserschwenkarm hingegen nicht im Schwenkbereich des Untersuchungsschwenkarms und der Untersuchungsschwenkarm nicht im Schwenkbereich des Laserschwenkarms angeordnet.

Dabei ist die zweite Achse des Untersuchungsschwenkarms so angeordnet, dass sie nichtparallel zur ersten Achse des Laserschwenkarms verläuft. Dies erlaubt einen kompakteren Aufbau als ein System mit einer zweiten Achse, die parallel zur ersten Achse verläuft.

Wenn also das ophthalmologische Therapiesystem einen Laserschwenkarm und einen Untersuchungsschwenkarm enthält, die beide am Gerätekopf schwenkbar befestigt sind, dann handelt es sich bei den beiden Schwenkarmen um zwei Einachsen-Armsysteme, deren Schwenkachsen vorzugsweise nicht parallel und vorzugsweise nicht senkrecht zueinander angeordnet sind. Weiterhin können beide Achsen in einer Ebene liegend angeordnet sein. Die Arbeitsposition des Untersuchungsschwenkarms und damit der Untersuchungseinrichtung wird dabei aus der Arbeitsposition des Laserschwenkarm und insbesondere der Position der Laseraustrittsöffnung abgeleitet.

Für die Lage der zweiten Achse, also der Schwenkachse des Untersuchungsschwenkarms, in Bezug auf die Lage der ersten Achse des Laserschwenkarms sind folgende Bereiche bevorzugt:
- ein Winkel der zweiten Achse zu einer senkrechten Ebene, die durch die erste Achse des Laserschwenkarmes verläuft, von 0° bis 20°, besonders bevorzugt von 0° bis 5°,
- ein Winkel der zweiten Achse zu einer horizontalen, also waagerechten, Ebene durch die erste Achse des Laserschwenkarmes, von 0° bis 50°, besonders bevorzugt von 20° bis 40°,
wobei eine horizontale Ebene eine Ebene ist, die parallel einer Bodenebene verläuft auf der das ophthalmologische Lasertherapiesystem - fest oder beweglich - angeordnet ist.

Weiterhin verläuft die zweite Achse bevorzugt durch einen Punkt, der sich 200mm bis 400mm oberhalb der Drehachse des Laserschwenkarmes, in y-Richtung 200mm bis 500mm hinter der Laseraustrittsöffnung für einen Laserschwenkarm in Arbeitsposition, d.h., auf den Gerätekopf zulaufend, und weniger als 100mm von der senkrechten Ebene durch die erste Achse, also die Drehachse des Laserschwenkarmes, entfernt befindet.

In einer weiteren grundlegenden Ausführungsform ist das erfindungsgemäße ophthalmologische Lasertherapiesystem dadurch gekennzeichnet, dass der Laserschwenkarm mit seiner zweiten Lasertherapieoptik und der Laseraustrittsöffnung von einem Schwenkarmgehäuse umfasst ist, das koaxial zum Laserschwenkarm am Gerätekopf separat schwenkbar befestigt ist. Laserschwenkarm und Schwenkarmgehäuse nutzen also dieselbe Schwenkachse, allerdings unabhängig voneinander.

Damit ist die zweite Lasertherapieoptik und die sich daran anschließende Laseraustrittsöffnung von dem Schwenkarmgehäuse schützend umgeben. Für die Laseraustrittsöffnung ist dabei eine entsprechende Öffnung im Schwenkarmgehäuse für den Austrittspunkt bzw. den Austrittsbereich des Laserstrahls vorgesehen.

Mit anderen Worten bildet das Schwenkarmgehäuse eine Außenhülle für den Laserschwenkarm, die aber separat koaxial zum Laserschwenkarm gelagert ist. Laserschwenkarm und Schwenkarmgehäuse werden in der Regel gleichzeitig um ihre jeweiligen Achsen geschwenkt, wenn es darum geht, den Laserschwenkarm in seinem Schwenkarmgehäuse von einer Ruheposition (auch Standby-Position genannt) in seine Arbeitsposition zu bewegen. Diese Ausführungsform stellt also eine echte "Arm-in-Arm"-Lösung dar.

Bevorzugt ist dabei der Laserschwenkarm um einen größeren Winkel als das Schwenkarmgehäuse schwenkbar. Ein Einziehen der Optik in das Schwenkarmgehäuse ist damit möglich:
Über das gemeinsame Schwenken von Laserschwenkarm und Schwenkarmgehäuse hinaus, beispielsweise von der Arbeitsposition in die Ruheposition oder umgekehrt, kann damit der Laserschwenkarm zunächst weiter in das Schwenkarmgehäuse "eingezogen" bzw. "versenkt" werden, um ihn während der Bewegung, aber auch in seiner Ruheposition bzw. in bestimmten Situationen in seiner Arbeitsposition, einen zusätzlichen Schutz zu gewähren. Andererseits kann der Laserschwenkarm etwas weiter aus dem Schwenkarmgehäuse "ausgefahren" werden, um beispielsweise den Koppelvorgang des Patientenauges an das ophthalmologische Lasertherapiesystem mittels eines Kontaktglases bzw. Patienteninterfaces einfacher zu gestalten. Zu diesem Zwecke lässt sich der Laserschwenkarm in positiver und/oder negativer Richtung um einen kleinen Betrag, der bevorzugt einen Wert zwischen 1° und 30°, insbesondere einen Wert zwischen 2° und 15°, und besonders bevorzugt einen Wert von ca. 3° annehmen kann, über die Endwinkelstellungen des Schwenkarmgehäuses hinaus schwenken.

Auch der Untersuchungsschwenkarm kann, analog dem Laserschwenkarm mit seinem Schwenkarmgehäuse, von einem koaxial gelagerten Untersuchungsschwenkarmgehäuse umfasst sein. Dabei sei hier mit dem allgemeinen Begriff Schwenkarmgehäuse immer das Gehäuse des Laserschwenkarms bezeichnet. Ist also ein Untersuchungsschwenkarm ebenfalls von einem Gehäuse umfasst, so ist dies näher bezeichnet als Untersuchungsschwenkarmgehäuse.

Damit sind für ein ophthalmologisches Lasertherapiesystem mit einem Laserschwenkarm und einem Untersuchungsschwenkarm bzgl. der Ausgestaltung der beiden Schwenkarme verschiedene Kombinationen möglich: Der Laserschwenkarm kann ein einfacher Schwenkarm sein, der am Gerätekopf angeordnet ist. Wesentlich bevorzugter ist jedoch ein Laserschwenkarm mit einem Schwenkarmgehäuse, wobei Laserschwenkarm und Schwenkarmgehäuse koaxial und separat voneinander schwenkbar angeordnet sind. Denn insbesondere für den Lasertherapieschritt, bei dem eine Laseraustrittsöffnung mit einem Auge des Patienten entweder mittels eines Kontaktglases bzw. Patienteninterfaces gekoppelt wird oder aber sich zumindest in nächster Nähe zum Auge befindet, sind die Vorteile einer solchen "Arm-in-Arm"-Lösung für die Patientensicherheit groß.

Auch für den Untersuchungsschwenkarm ist es möglich, dass er entweder als einfacher Untersuchungsschwenkarm am Gerätekopf des ophthalmologischen Lasertherapiesystems angeordnet ist, oder aber von einem Untersuchungsschwenkarmgehäuse umgeben ist. Dabei wird die Entscheidung danach getroffen, welche Untersuchungseinrichtung der Untersuchungsschwenkarm umfasst, sowie bei Vorliegen mehrerer Untersuchungseinrichtungen, wie diese bzw. einzelne Elemente dieser zueinander angeordnet sind. Laserschwenkarm und Untersuchungsschwenkarm können dabei in jeglicher Kombination (beide mit Arm-in-Arm-Lösung, nur einer von beiden mit "Arm-in-Arm"-Lösung oder beide als einfache Schwenkarme) eingesetzt werden. Aufgrund des Aufbaus der Arm-in-Arm-Lösung erfordert diese nämlich keinen signifikanten zusätzlichen Platz und schränkt auch die Anordnung der Achsen der beiden Arme zueinander nicht ein.

Je nach Anwendung und insbesondere je nach Untersuchungsgerät und dessen räumlichen Möglichkeiten kann dabei Sorge getragen werden, dass eine Arbeitsposition des Laserschwenkarms eine Ruheposition des Untersuchungsschwenkarms bedingt und umgekehrt. Gegebenenfalls, wie beispielsweise beim Einsatz eines Operationsmikroskops am Untersuchungsschwenkarm, ist dies ein Sicherheitsaspekt, so dass durch ein Untersagen von gleichzeitigen Arbeitspositionen des Laserschwenkarms mit seinem Schwenkarmgehäuse und des Untersuchungsschwenkarms ein kollisionsfreies Arbeiten garantiert wird.

Weiterhin bevorzugt weisen der Laserschwenkarm sowie das Schwenkarmgehäuse jeweils eine separate Gewichtsausgleichsvorrichtung auf.

Dies ermöglicht eine sehr reibungsarme Gewichtsausgleichsmechanik, die eine Bewegung mit geringer Kraft ermöglicht, also etwa einer Kraft kleiner 3N für den Laserschwenkarm. Damit ist es ausgeschlossen, dass beim Koppeln des Patientenauges an das ophthalmologische Lasertherapiesystem mittels eines Kontaktglases bzw. Patienteninterfaces Quetschungen entstehen.

Wesentlich ist hier, dass die separaten Gewichtsausgleiche in ihren Eigenschaften an ihre jeweilige Funktion angepasst sind: Der Gewichtsausgleich für den Laserschwenkarm ist sehr präzise und reibungsarm, um eine Kraft auf das Auge auf die genannten 3 N zu begrenzen. Hingegen hat der Gewichtsausgleich des Schwenkarmgehäuses mehr Reibung und eine größere Auflagekraft in der Endposition, damit ungewollte Auslenkungen des Arms vermieden werden. Hier muss nur eine Quetschung des ganzen Kopfes verhindert werden, wofür größere Kräfte zulässig sind: Im Gesicht beträgt die maximal zulässige Kraft ca. 65N. Für das Schwenkarmgehäuse wird deshalb eine Bewegung mit einer Kraft von kleiner als 65N ermöglicht.

Zur Quetschvermeidung gehört zudem, dass das Trägheitsmoment des Laserschwenkarms und die maximale Annäherungsgeschwindigkeit an das Patientenauge, die von der Bewegung einer Patientenlagerungseinrichtung, beispielsweise einer Patientenliege, oder von der Bewegung des Gerätekopfs ausgeht, in einem bestimmten Verhältnis stehen: Ein Trägheitsmoment von 1,9kgm² und eine statische Auflagekraft von 1,5N ergeben bei einer Annäherungsgeschwindigkeit von 30mm/s eine Maximalkraft von ~3N am Auge. Wird das Trägheitsmoment nun verkleinert und nimmt einen bevorzugten Wert von 1,5kgm² an, dann kann die maximale Annäherungsgeschwindigkeit etwas erhöht werden.

Wird der Gewichtsausgleich nun beispielsweise mit Hilfe einer Gewichtsausgleichsvorrichtung erzeugt, die eine Gasfeder enthält, so ermöglicht dies eine kompakte Bauweise und die Integration einer Dämpfung zum Vermeiden von hohen Geschwindigkeiten sowie zum Abbremsen in den Endstellungen. Sie kommt allerdings vor allem für den Gewichtsausgleich des Schwenkarmgehäuses in Frage, da sie wegen der Gasdichtungen nicht reibungsarm ist.

Bei der Überwachung der in der Gewichtsausgleichsvorrichtung enthaltenen Gasfeder wird die Spannkraft der Gasfeder in der Aufhängung überprüft, indem diese geringfügig beweglich mit einem Schaltelement, beispielsweise einem Schalter oder einer Lichtschranke, verbunden ist, und eine Sensorfeder mit der Aufhängung verbunden ist, die bei einer Normkraft der Gasfeder bis zu einem Anschlag gedehnt wird. Ist die Kraft der Gasfeder hingegen zu klein, bewegt die Sensorfeder die Aufhängung der Gasfeder zurück und das Schaltelement wird geöffnet. Ist das Schaltelement in einer geöffneten Stellung, wird das Herunterschwenken des Laserschwenkarms bzw. des Schwenkarmgehäuses aus einer Ruheposition in eine Arbeitsposition nicht freigegeben.

Auch der Untersuchungsschwenkarm enthält in der Regel eine Gewichtsausgleichsvorrichtung, bei Vorliegen eines Untersuchungsschwenkarmgehäuses kann auch dieses eine separate Gewichtsausgleichsvorrichtung aufweisen. Damit wird auch hier ein Gewichtsausgleich erzielt, der erhöhten Sicherheitsanforderungen genügt, und es trotzdem erlaubt, kleine Motoren einzusetzen. Die Arme sind dabei jederzeit und ohne großen Kraftaufwand manuell bewegbar.

Eine solche Gewichtsausgleichsvorrichtung kann auch in diesem Fall mit einer Gasfeder und einer entsprechenden oben beschriebenen Überwachung ausgeführt sein.

Zudem können natürlich auch weitere Untersuchungs- oder Therapieschwenkarme nach denselben hier beschriebenen Prinzipien am Gerätekopf des ophthalmologischen Lasertherapiesystems schwenkbar befestigt sein. Dabei sind bevorzugt bis zu fünf Schwenkarmen mit jeweils eigener Schwenkachse denkbar. Auch diese weiteren Schwenkarme, für die das Schwenken in verschiedene Stellungen sehr unterschiedliche Kraftmomente erfordert, weisen dann jeweils entsprechende Gewichtsausgleichsvorrichtungen auf. Umfasst der jeweilige Schwenkarm ein Gehäuse, so ist dessen Gewichtsausgleich mit einer vom Schwenkarm unabhängigen Gewichtsausgleichsvorrichtung realisiert.

Besonders vorteilhaft ist des Weiteren ein ophthalmologisches Lasertherapiesystem, dessen Gerätekopf auf der Gerätebasis auch in z-Richtung verfahrbar ist. Dies erlaubt es, den Abstand zwischen Gerätebasis und Gerätekopf zu variieren. Durch diese Abstandsänderung wird die Arbeitshöhe, also die Höhe der Laseraustrittsöffnung, auf einfache Art und Weise verändert. Bei einer bevorzugten Anordnung der Laserquelle im Gerätekopf wird bei einem Verfahren des Gerätekopfes auf der Gerätebasis in z-Richtung die Laserquelle mitverschoben, so dass hier keinerlei Ausgleich erforderlich ist.

In einer Ausgestaltung umfasst das ophthalmologische Lasertherapiesystem eine Vorpositionierungseinrichtung zur Vorpositionierung des Gerätekopfes.

Die Anordnung der Vorpositionierungseinrichtung ist so gewählt, dass ein Objekt in einem Arbeitsvolumen des Laserstrahls, wenn sich der Laserschwenkarm in einer Arbeitsposition befindet, parallaxefrei beobachtet werden kann. Das Arbeitsvolumen ist dabei der mögliche Wirkbereich des fokussierten Laserstrahls bei einem in Arbeitsposition angeordneten Laserschwenkarm.

Vorzugsweise enthält diese Vorpositionierungseinrichtung eine Kamera. Diese ist aus den hier genannten Gründen vorteilhaft am Gerätekopf fixiert. Das ermöglicht ein schnelles und einfaches Vorpositionieren des Gerätekopfes mittels der Transversalbewegung in der x-y-Ebene in einer y-Richtung, bzw. wenn eine Kamera verwendet wird, die zusätzlich Tiefeninformationen liefert, auch in einer x-Richtung, und ggf. auch in der z-Richtung in Bezug auf die Gerätebasis.

In einer weiteren Ausgestaltung enthalten der Laserschwenkarm und/oder das Schwenkarmgehäuse des ophthalmologischen Lasertherapiesystems mindestens eine Detektionseinrichtung. Eine solche Detektionsvorrichtung dient dem Erfassen von Strukturen in einem Arbeitsvolumen des Laserschwenkarms. Insbesondere kann es sich bei der Detektionseinrichtung um eine Videomikroskop-Vorrichtung oder eine OCT-Vorrichtung handeln.

Eine Detektionseinrichtung, die am bzw. im Laserschwenkarm und/oder dem Schwenkarmgehäuse des ophthalmologischen Lasertherapiesystems enthalten ist, macht den Einsatz eines klassischen Operationsmikroskops, das üblicherweise in vielen Lasertherapieverfahren in verschiedenen Arbeitsschritten genutzt wird, fakultativ.

Die Detektionseinrichtung kann als integrierte Detektionseinrichtung ausgestaltet sein, d.h., die Strahlführungsmittel und insbesondere die Lasertherapieoptik des Laserschwenkarms mitnutzen. Die Detektionseinrichtung kann aber auch als unabhängige Detektionseinrichtung ausgestaltet sein, die allenfalls ebenfalls durch die Laseraustrittsöffnung austritt und von da auf ein Arbeitsvolumen beispielsweise in einem Gewebe eines Patientenauges gerichtet ist. Ein gleicher Austrittsort von einer zur Therapie verwendeten Laserstrahlung und einer Detektionsstrahlung bietet Vorteile bzgl. der Kalibrierung der Strahlung, insbesondere ihrer Fokusse, zueinander.

Dennoch ist es auch möglich, mit einer Detektionseinrichtung zu arbeiten, die Teil des Laserschwenkarms und/oder des Schwenkarmgehäuses des ophthalmologischen Lasertherapiesystems ist, deren Strahlengang bzw. Wellenverlauf jedoch völlig unabhängig zu dem der Laserstrahlung ist, solange eine Beziehung zwischen der Laserstrahlung und der Strahlung bzw. den Wellen, die von der Detektionseinrichtung genutzt werden, in Bezug auf deren Einsatzort etabliert ist.

Weitere Beispiele von Detektionseinrichtungen, die der Laserschwenkarm und/oder das Schwenkarmgehäuse enthalten kann, sind Ultraschall-Sensoren, Mikrowellen-Sensoren, eine optische Kohärenz-Tomographie-Vorrichtung (OCT), Interferometer, Wellenfrontsensoren, Videosensoren oder ein klassisches Mikroskop in einer sehr kompakten Ausführung.

Weiterhin ist es vorteilhaft, wenn eine Eingabe- und/oder Ausgabevorrichtung am Schwenkarmgehäuse des ophthalmologischen Lasertherapiesystems beweglich befestigt ist. Eine solche Eingabe- und/oder Ausgabevorrichtung kann insbesondere durch einen Therapiebildschirm dargestellt sein, wobei dieser vorzugsweise zu Eingabezwecken als Touch-Screen ausgebildet ist und die Ausgabe in Textform und/oder graphischer Form möglich ist.

Bevorzugt ist die Untersuchungseinrichtung eines Untersuchungsschwenkarms des ophthalmologischen Lasertherapiesystems ein Operationsmikroskop (OPMI). Operationsmikroskope werden in entsprechenden Therapieverfahren am Auge von einer großen Mehrheit der Operateure eingesetzt, insbesondere, um den Verlauf eines Therapieverfahrens zu beobachten, Ergebnisse visuell zu verifizieren oder im Therapieverfahren enthaltene manuelle Schritte präzise durchführen zu können.

Von besonderem Interesse ist des Weiteren eine Ausführungsform des ophthalmologischen Lasertherapiesystems, das eine Eingabe und/oder Ausgabevorrichtung oder eine Untersuchungseinrichtung enthält, die dadurch gekennzeichnet ist, dass die Eingabe- und/oder Ausgabevorrichtung um eine zur ersten Achse des Schwenkarmgehäuses und/oder die Untersuchungseinrichtung um eine zur zweiten Achse des Untersuchungsschwenkarms parallele Achse drehbar und über eine Koppelvorrichtung an die Bewegung des Schwenkarmgehäuses oder des Untersuchungsschwenkarms gekoppelt ist. Eine solche Koppelvorrichtung ist beispielsweise eine Koppelstange oder ein Seilzug.

Damit verbleibt sowohl die Eingabe- und/oder Ausgabevorrichtung, also beispielsweise ein Therapiebildschirm, wie auch die Untersuchungseinrichtung, also beispielsweise ein Operationsmikroskop, bei der Schwenkbewegung des jeweiligen Arms an der sie beweglich befestigt sind, immer in einer Arbeitslage. Anzeigen auf einer Eingabe- und/oder Ausgabevorrichtung werden so beispielsweise immer in korrekter, für einen Bediener bzw. Operateur bevorzugten Ausrichtung angezeigt, Untersuchungseinrichtungen oder Komponenten, die beim Verkippen beschädigt oder dejustiert werden, können auf diese Art und Weise immer in einer aufrechten Lager gehalten werden, auch wenn sich der jeweilige Schwenkarm in einer Ruheposition befindet.

Bevorzugt enthält die erste und/oder die zweite Lasertherapieoptik des ophthalmologischen Lasertherapiesystems ein Scansystem mit mindestens zwei Scannern, die bewegliche Strahlführungsmittel sind, derart, dass die erste Achse des Laserschwenkarms in der Strahlachse zwischen den Scannern angeordnet ist.

Damit ist das optische System beim Schwenken unempfindlich gegen kleine Verdrehungen. Zur Sicherheit wird jedoch nur in der Arbeitsposition gearbeitet, d.h. nur in der Arbeitsposition wird der Laserstrahl zur therapeutischen Behandlung, die hier auch eine chirurgische Behandlung einschließt, genutzt. In anderen Positionen als der Arbeitsposition besteht die Gefahr, dass die Ablenkachsen der Scanner signifikant gegeneinander verdreht sind.

Weiterhin enthält eine vorteilhafte Ausführungsform des ophthalmologischen Lasertherapiesystems eine Lageregelungsvorrichtung zur Regelung der Lage zwischen dem Laserschwenkarm und/oder dem Untersuchungsschwenkarm in Arbeitsposition und einem Patientenauge. Sie ist eingerichtet, die Lage des Gerätekopfes der Position eines Patientenauges nachzuführen.

Die Lageregelungsvorrichtung umfasst bevorzugt einen Kraftsensor und/oder eine Lichtschranke. Ein Kraftsensor kann hierfür beispielsweise so angeordnet sein, dass die zweite Lasertherapieoptik bzw. der gesamte Laserschwenkarm auf dem Kraftsensor liegt, wenn sich der Laserschwenkarm in Arbeitsposition befindet.

Eine Druckkraft des Patientenauges gegen die zweite Lasertherapieoptik bzw. die Laseraustrittsöffnung, ggf. über ein Kontaktglas oder ein Patienteninterface, entlastet den Kraftsensor. Ist dies der Fall, dann wird die Position des Gerätekopfes so verändert dass sich der Laserschwenkarm in Arbeitsposition vom Patienten entfernt.

Wird hingegen eine höhere Druckkraft auf den Kraftsensor dadurch ausgeübt, dass das Patientenauge im fixierten ("angedockten") Zustand, also beispielsweise in einem über ein Kontaktglas oder ein Patienteninterface mit der Laseraustrittsöffnung verbundenem Zustand, am Laserschwenkarm zieht, so wird die Position des Gerätekopfes so verändert, dass der Laserschwenkarm in Arbeitsposition dem Patientenauge entgegenkommt. Die Veränderung der Position des Gerätekopfes erfolgt durch entsprechende Translationsbewegung relativ zur Gerätebasis.

Die reguläre Lageregelung erfolgt also in einer bevorzugten Ausführungsform über eine Nachführung des Gerätekopfes in z-Richtung. Dabei muss der entsprechende Arbeitsschritt, insbesondere der Lasertherapieschritt, nicht unterbrochen werden. Sind dabei jedoch definierte untere bzw. obere Grenzwerte erreicht, oder werden Bewegungsmuster detektiert, die als kritisch eingeschätzt werden, so wird das Verfahren gestoppt, ggf. also die Lasereinrichtung ausgeschaltet: Dies ist beispielsweise der Fall, wenn sich das Patientenauge so weit nach unten entfernt, dass die Fixierung des Patientenauges an der Laseraustrittsöffnung mittels des Kontaktglases bzw. des Patienteninterfaces nicht mehr gewährleistet werden kann. Dies ist beispielsweise auch der Fall, wenn sich das Patientenauge so weit oder aber so schnell nach oben bewegt, dass eine Kompensation durch Nachführung des Gerätekopfes in z-Richtung nicht mehr möglich ist, und der Laserschwenkarm durch eine entsprechende Druckkraft des Patientenauges nach oben geschwenkt wird, wobei hier daran erinnert wird, dass dafür nur eine ungefährliche Druckkraft von kleiner 3N erforderlich ist, so dass das Patientenauge bei dieser Aktion nicht geschädigt wird. Ist der Laserschwenkarm von einem Schwenkarmgehäuse umfasst, dann wird der zunächst der Laserschwenkarm mit der für das Auge ungefährlichen Druckkraft von kleiner 3N in das Schwenkarmgehäuse "versenkt", so dass dann das Schwenkarmgehäuse auf dem Gesicht zum Aufliegen kommt. Das Schwenkarmgehäuse kann dann im Weiteren zusammen mit dem Laserschwenkarm mit einer für das Gesicht ungefährlichen Druckkraft von kleiner 65N nach oben geschwenkt werden.

Eine solche Lösung vereinfacht erheblich die Konstruktion einer Patientenliege oder ganz allgemein einer Patientenlagerungseinrichtung, die zur Ausführung einer Lasertherapie an einem Patientenauge genutzt wird, um den Patienten unter den Schwenkarmen des ophthalmologischen Lasertherapiesystems in Arbeitsposition zu platzieren. Ohne eine solche Lageregelungsvorrichtung und das hier beschriebene Sicherheitskonzept müsste die Patientenliege selbst auf das Erreichen kritischer Grenzwerte reagieren: Neben dem Abschalten des Lasertherapiesystems müsste auch die Bewegung der Patientenliege in die kritische Richtung sofort abgestellt werden. Wird jedoch eine Lageregelungsvorrichtung und das Sicherheitskonzept wie hier beschrieben eingesetzt, kann auf derartige Mechanismen an der Patientenliege verzichtet werden: Die Patientenliege muss somit nicht mehr in das ophthalmologische Lasertherapiesystem bzw. in dessen Funktionen integriert werden. Damit kann eine beliebige Patientenlagerungsvorrichtung verwendet werden.

Ein ophthalmologisches Lasertherapieverfahren mit einer erfindungsgemäßen ophthalmologischen Lasertherapiesystem umfasst die folgenden Schritte:
- Ein Patient wird auf einer Patientenlagerungsvorrichtung, bevorzugt auf einer Patientenliege, neben einem oben beschriebenen erfindungsgemäßen ophthalmologischen Lasertherapiesystem, dessen Schwenkarme sich in Ruheposition befinden, positioniert. Die Patientenlagerungsvorrichtung kann dabei einfachster Ausführung sein. Sie bedarf keiner Positionierungsvorrichtungen, wie es ansonsten erforderlich ist, wenn über die Position der Patientenlagerungsvorrichtung der Patient von einem Arbeitsvolumen in ein Untersuchungsvolumen oder aber in ein anderes Arbeitsvolumen verbracht werden muss. So kann sie beispielsweise rein manuell verstellbar sein.

Der Gerätekopf wird - bevorzugt mittels der Vorpositionierungseinrichtung, beispielsweise einer Kamera - vorpositioniert. Hierzu wird beispielsweise ein überlagertes "Sinnbild", also eine vorausgesagte Position, des heruntergeschwenkten Laserschwenkarmes auf einem Therapiebildschirm sichtbar gemacht, auf dem ein Bild, das die Vorpositionierungseinrichtung erzeugt hat, angezeigt wird. Dadurch kann der Gerätekopf lateral in y-Richtung, bei einer Kamera, die Tiefeninformationen liefert, auch in x-Richtung, und vorzugsweise auch in z-Richtung, also in der Höhe, vorpositioniert werden.

Der Laserschwenkarm wird anschließend in eine Arbeitsposition geschwenkt, und weiter über dem Patientenauge positioniert, bevorzugt mittels einer an einem Schwenkarmgehäuse enthaltenen Videomikroskop-Vorrichtung.

Ist der Laserschwenkarm über dem Patientenauge positioniert, wird der Lasertherapieschritt durchgeführt. Dieser Schritt kann beispielsweise von Bediener bzw. Operateur mittels eines Fußschalters gestartet werden.

Ist der Lasertherapieschritt beendet, wird der Gerätekopf vorzugsweise zunächst hochgefahren, sofern hierzu eine entsprechende Möglichkeit der Verfahrbarkeit des Gerätekopfes auf der Gerätebasis in z-Richtung gegeben ist. Der Laserschwenkarm wird hernach in die Ruheposition geschwenkt.

Gegebenenfalls können diese Schritte über einem zweiten Patientenauge wiederholt werden.

Ein bevorzugtes ophthalmologisches Lasertherapieverfahren enthält des Weiteren folgende Schritte:
Ein Schwenkarmgehäuse, das den Laserschwenkarm umfasst, wird synchron mit dem Laserschwenkarm in eine Arbeitsposition geschwenkt. Dabei ist die Laseraustrittsöffnung bevorzugt noch in das Schwenkarmgehäuse eingezogen.

Vor der Positionierung über dem Patientenauge wird der Laserschwenkarm weiter aus dem Schwenkarmgehäuse herausgeschwenkt. Die zweite Lasertherapieoptik und die Laseraustrittsöffnung bewegen sich dabei mit einer durch einen Gewichtsausgleich des Laserschwenkarms eingestellten Kraft.

Nach der Durchführung des Lasertherapieschritts wird der Laserschwenkarm in das Schwenkarmgehäuse eingeschwenkt, d.h., die Laseraustrittsöffnung wird wieder in das Schwenkarmgehäuse eingefahren.

Das Schwenkarmgehäuse wird synchron mit dem Laserschwenkarm in die Ruheposition geschwenkt. Dabei erfolgt der Gewichtsausgleich für den Laserschwenkarm sowie für das Schwenkarmgehäuse bevorzugt unabhängig voneinander.

Das beschriebene ophthalmologische Lasertherapieverfahren kann bevorzugt weiterhin folgende Schritte enthalten:
- Ansetzen eines Kontaktglases oder eines Patienteninterfaces an die Laseraustrittsöffnung (9) und Festhalten des Kontaktglases oder des Patienteninterfaces an der Laseraustrittsöffnung (9) mittels Unterdruck, wobei der Unterdruck ein- bzw. ausgeschaltet wird durch den Druck des Kontaktglases oder des Patienteninterfaces gegen die Laseraustrittsöffnung (9).
- Ansaugen des Patientenauges, vorzugsweise unterstützt durch eine Detektionseinrichtung (13), insbesondere durch eine Videomikroskop-Vorrichtung, unter weiteren Positionieren des Gerätekopfes (2)
- Lösung der Ansaugung des Kontaktglases oder des Patienteninterfaces nach der Durchführung des Lasertherapieschrittes.

Ein Kontaktglas oder ein anderes Patienteninterface wird an die Laseraustrittsöffnung mittels Unterdruck angesetzt, wobei der Unterdruck ein- bzw. ausgeschaltet wird durch den Druck des Kontaktglases oder des Patienteninterfaces gegen die Laseraustrittsöffnung. Das Ansetzen des Kontaktglases oder des Patienteninterfaces an die Laseraustrittsöffnung erfolgt dabei in der Regel im noch eingefahrenen Zustand des Laserschwenkarms, damit der Druck des Kontaktglases oder Patienteninterfaces zum Schaltvorgang führt:
In der Regel wird aus Sicherheitsgründen der Laserschwenkarm im herausgefahrenen Zustand niemals blockiert, so dass er jederzeit bei Druck in entgegengesetzte Richtung wieder in den Laserschwenkarm hineingedrückt wird und somit das für eine Quetschungsvermeidung notwendige Spiel aufweist. Für den Schaltvorgang ist jedoch ein Widerstand nötig.

In einer besonderen Ausführungsform kann ein solcher Widerstand aber auch dadurch erreicht werden, dass der Laserschwenkarm im herausgefahrenen Zustand eine Blockiermöglichkeit aufweist, die jedoch wiederum durch eine Sicherheitsschaltung nach dem Ansetzen des Kontaktglases oder Patienteninterfaces und vor dem Andocken des Kontaktglases oder Patienteninterfaces an das Patientenauge außer Betrieb gesetzt wird, so dass der Laserschwenkarm beim oder nach dem Andocken des Patientenauges jederzeit auf Druck nachgibt und wieder einfährt.

Damit wird ein Ansetzen des Kontaktglases bzw. Patienteninterfaces für solche ophthalmologischen Lasertherapiesysteme, deren Schwenkarmgehäuse aufgrund besonderer Aufbauten einen großen Abstand zum Laserschwenkarm haben und ein Ansetzen des Kontaktglases bzw. Patienteninterfaces mit eingefahrenem Laserschwenkarm sehr unkomfortabel wäre, auch nach dem Herausschwenken des Laserschwenkarms aus dem Schwenkarmgehäuse möglich.

Ein Kontaktglas ist dabei im Sinne dieser Beschreibung eine in der Regel an die Wölbung der Hornhaut des Auges angepasstes gewölbtes optisches Element - i.d.R. ein Linsenelement - oder aber oder ein auf Augenseite flaches und auf Seiten der Laseraustrittsöffnung zumeist konvex gewölbtes optisches Element, das auf dem Patientenauge aufliegt, und das eine feste Relativbeziehung zwischen der Laseraustrittsöffnung und dem Auge des Patienten herstellen soll, das Patientenauge also am ophthalmologischen Lasertherapiesystem temporär fixieren soll.

Ein Patienteninterface erfüllt dieselbe Funktion. Allerdings liegt in diesem Fall das optische Element nicht auf dem Patientenauge auf. Das Auge wird demnach von dem optischen Element auch nicht verformt. Vielmehr wird das Patienteninterface, das in der Regel eine konische Form aufweist und über einen äußeren Ring auf dem Auge - zumeist mittels eines Saugrings - fixiert wird, mit einer Flüssigkeit gefüllt, in die das optische Element mit seiner augenseitigen Oberfläche eingetaucht wird.

Dadurch wird die Hornhaut des Patientenauges nicht bzw. nur am äußeren Rand verformt.

Das Patientenauge wird, vorzugsweise unterstützt durch eine Detektionseinrichtung, insbesondere eine Videomikroskop-Vorrichtung, durch weiteres Positionieren des Gerätekopfes angesaugt. Damit ist das Patientenauge nun vollständig am ophthalmologischen Lasertherapiesystem fixiert.

Nach Beendigung der Durchführung des Lasertherapieschritts wird die Ansaugung des Kontaktglases oder des Patienteninterfaces gelöst, beispielsweise indem der Unterdruck beseitigt wird.

Das beschriebene ophthalmologische Lasertherapieverfahren kann weiterhin vorteilhaft folgende Schritte enthalten:
- Planung der Behandlungsparameter an einem Planungsbildschirm (31) vor dem Platzieren des Patienten auf der Patientenlagerungsvorrichtung (500), und
- Übergabe an eine Eingabe- und/oder Ausgabevorrichtung, insbesondere einen Therapiebildschirm (12).

Vor dem Platzieren des Patienten auf der Patientenlagerungsvorrichtung, werden also die Behandlungsparameter an einem Planungsbildschirm geplant und anschließend an eine am Schwenkarmgehäuse eingeordneten Eingabe- und/oder Ausgabevorrichtung, also beispielsweise einen Therapiebildschirm, übergeben.

Ein solcher Therapiebildschirm, der Eingabe- und Ausgabevorrichtung darstellt, ist bevorzugt ein Touch-Screen. Dabei hat der Therapiebildschirm gegenüber dem Planungsbildschirm einen vereinfachten Aufbau. In der graphischen Gestaltung des Bildschirms kann so beispielsweise nur eine reduzierte Anzahl von Parametern angegeben sein, was den Therapiebildschirm gegenüber dem Planungsbildschirm übersichtlicher macht. Damit können Fehler im Verlaufe des ophthalmologischen Lasertherapieverfahrens vermieden werden.

Das beschriebene ophthalmologische Lasertherapieverfahren kann weiterhin folgende Schritte enthalten:
- Schwenken eines Untersuchungsschwenkarms (14) von einer Ruheposition in eine Arbeitsposition nach dem Vorpositionieren des Gerätekopfes (1) und/oder nach dem Schwenken des Laserschwenkarms (3) von einer Arbeitsposition in eine Ruheposition nach der Durchführung des Lasertherapieschritts
- Durchführung weitere Arbeitsschritte unter Zuhilfenahme einer Untersuchungseinrichtung (15).

Nicht zuletzt ist es von großer Hilfe, wenn nach dem Vorpositionieren des Gerätekopfes und/oder nach dem Schwenken des Laserschwenkarms von einer Arbeitsposition in eine Ruheposition nach der Durchführung des Lasertherapieschritts ein Untersuchungsschwenkarm von einer Ruheposition in eine Arbeitsposition geschwenkt wird, und weitere Arbeitsschritte unter Zuhilfenahme einer Untersuchungseinrichtung des Untersuchungsschwenkarms durchgeführt werden.

Die erfindungsgemäße Lösung der Aufgabe in ihren Ausgestaltungen vermeidet also den Nachteil des Umpositionierens des Patienten durch eine Anordnung eines Laserschwenkarms, der über das Patientenauge geschwenkt und positioniert werden kann, und ggf. die Anordnung eines weiteren Untersuchungsschwenkarms, der beispielsweise als Untersuchungseinrichtung ein Operationsmikroskop enthalten kann, in einer definiert beweglichen Anordnung der beiden Schwenkarme zueinander. Diese Beweglichkeit ermöglicht ein wechselweises Positionieren der beiden Schwenkarme, und damit beispielsweise des Fokus des Laserstrahls und des Operationsmikroskops, auf denselben Arbeitspunkt in einem Arbeits- bzw. Untersuchungsvolumen (in dem sich das Patientenauge befindet), beispielsweise zum Umschalten zwischen Lasertherapie und Lentikelextraktion bei einer SMILE-Behandlung.

Zudem kann die empfindliche Lasertherapieoptik des Laserschwenkarms durch ein Arm-in-Arm-Prinzip mittels eines koaxial gelagerten Schwenkarmgehäuses geschützt werden. Laserschwenkarm und Schwenkarmgehäuse werden dabei unabhängig voneinander gelagert und gewichtskompensiert.

Der Umfang der vorliegenden Erfindung wird durch die Ansprüche definiert.

Die vorliegende Erfindung soll nun anhand von Ausführungsbeispielen erläutert werden. Es zeigt:
- die Fig. 1a bis 1d: ein erfindungsgemäßes ophthalmologisches Lasertherapiesystem, wobei die Fig. 1a verschiedene Teilbereiche aufzeigt, deren Ausgestaltungen zu verschiedenen Ausführungsformen des erfindungsgemäßen ophthalmologischen Lasertherapiesystems führen und in der Fig. 1b ein Standby-Modus, in der Fig. 1c ein Lasertherapiemodus und in der Fig. 1d ein Untersuchungsmodus unter Einsatz eines Operationsmikroskops einer ersten Ausführungsform des erfindungsgemäßen ophthalmologischen Lasertherapiesystems dargestellt ist.
- die Fig. 2a und 2b: das Arm-in-Arm Prinzip von Laserschwenkarm und Schwenkarmgehäuse in einer bevorzugten Ausführungsform eines erfindungsgemäßen ophthalmologischen Lasertherapiesystems
- die Fig. 3a und 3b: eine schematische Darstellung einer Vorrichtung zur Überwachung der Funktion der Feder einer Gewichtsausgleichsvorrichtung.
- die Fig. 4: die Koppelmechanik des Therapiebildschirms an die Bewegung des Schwenkarmgehäuses
- die Fig. 5: die Anordnung einzelner Elemente eines erfindungsgemäßen ophthalmologischen Lasertherapiesystems in Ruheposition mit eingezogener Optik

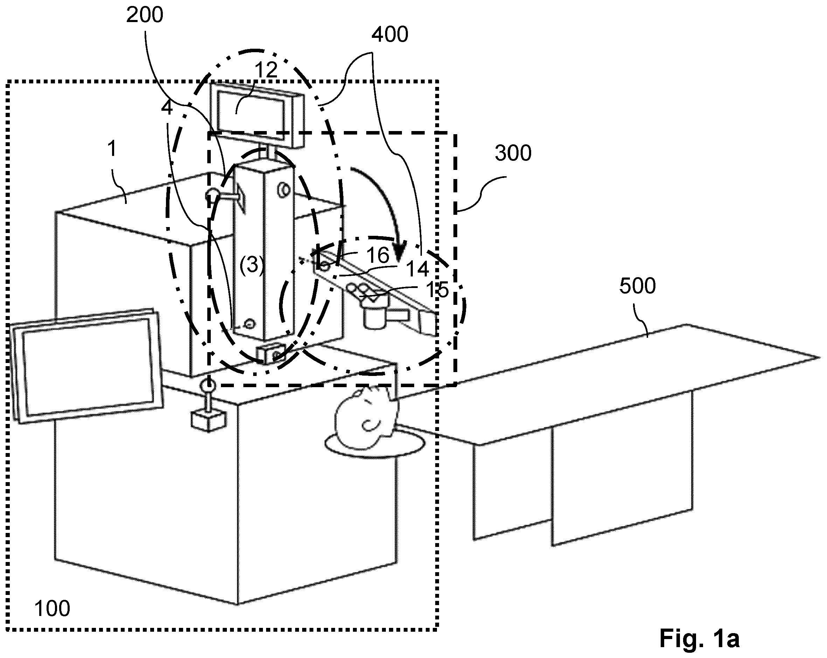

**In** der Fig. 1a sind zunächst verschiedene Teilbereiche aufzeigt, die zu einer Lösung der Aufgabe dieser Erfindung beitragen. Unterschiedliche Ausgestaltungen dieser Teilbereiche führen zu verschiedenen Ausführungsformen des erfindungsgemäßen ophthalmologischen Lasertherapiesystems 100.

Dabei sei zunächst bemerkt, dass die Patientenliege 500 nicht Teil des erfindungsgemäßen ophthalmologischen Lasertherapiesystems 100 ist, sondern in den Figuren lediglich zu einem besseren Verständnis der Funktion und insbesondere des Arbeitsbereichs von erfindungsgemäßen ophthalmologischen Lasertherapiesystemen 100 dargestellt ist. Aus der Funktion dieser ophthalmologischen Lasertherapiesysteme ist jedoch der Vorteil nachvollziehbar, an dieser Stelle Patientenliegen 500 einer sehr einfachen Ausführungsform einsetzen zu können.

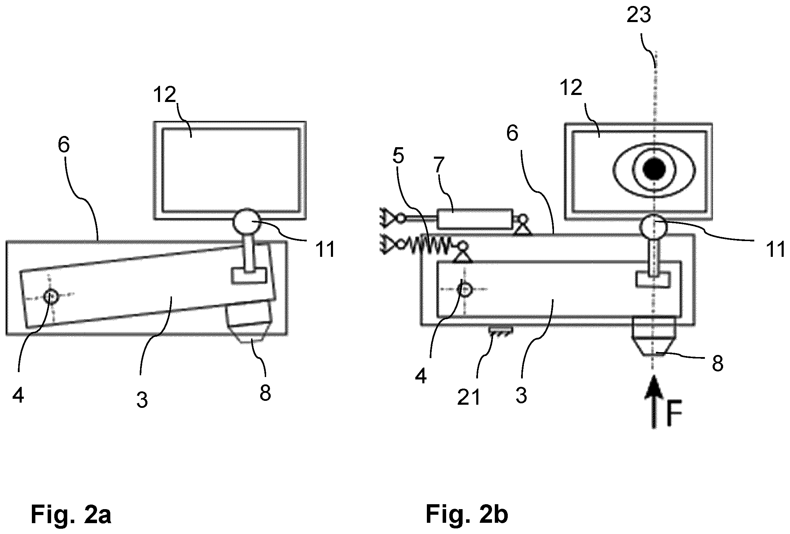

Erfindungsgemäße ophthalmologische Lasertherapiesysteme 100 zeichnen sich aus durch einen am Gerätekopf 1 um eine horizontale Achse 4 schwenkbar befestigten Laserschwenkarm 3, der zwischen einer Ruheposition und einer Arbeitsposition hin- und hergeschwenkt werden kann. Da dieser Laserschwenkarm 3 für die Lasertherapie am Patientenauge über den Patienten geschwenkt wird, in Schritten jedoch, in denen der Laserschwenkarm 3 nicht benötigt wird, in eine Ruheposition zurückverbracht werden kann, um den Raum über der Arbeitsposition anderweitig zu nutzen, wird der Laserschwenkarm 3 einerseits dadurch geschützt, dass der Laserschwenkarm 3 von einem Schwenkarmgehäuse 6 umfasst ist, das koaxial zum Laserschwenkarm 3 am Gerätekopf 1 schwenkbar befestigt ist. Dies ist das "Arm-in-Arm"-Prinzip 200. Neben einem mechanischen Schutz für den Laserschwenkarm 3 selbst bietet das "Arm-in-Arm"-Prinzip des Weiteren den Vorteil von unabhängigen Gewichtsausgleichsvorrichtungen für Laserschwenkarm 3 und Schwenkarmgehäuse 6, sowie insbesondere auch die Möglichkeit, Gewichte von Aufbauten zu verteilen. Details dieses Arm-in-Arm-Prinzips 200 zeigen die Fig. 2a und 2b.

Wird neben dem Laserschwenkarm 3 des Weiteren Gebrauch gemacht von einem oder mehreren weiteren selbständigen Untersuchungsschwenkarmen 14, so sind diese jeweils um eine Achse schwenkbaren Armsysteme so zueinander angeordnet, dass sämtliche Arbeitsschritte einer Lasertherapie am Auge eines Patienten so ausgeführt werden können, dass der Wirkungsort aller Arbeitsschritte unter Zuhilfenahme des Laserschwenkarms 3 bzw. eines oder mehrerer Untersuchungsschwenkarme 6 immer fest bleibt, die Position des Patientenauges während der gesamten Lasertherapie also nicht verändert werden muss. Die Position des Patientenauges im ersten Arbeitsschritt bestimmt die Position aller nachfolgenden Arbeitsschritte und damit den Wirkungsort ihrer jeweils notwendigen Einrichtungen an den verschiedenen Schwenkarmen 3, 14. Dies wird ermöglichst durch eine spezielle Anordnung 300 der Schwenkachsen 4, 16 der verschiedenen Schwenkarme 3, 14 zueinander am Gerätekopf 1 des ophthalmologischen Lasertherapiesystems 100.

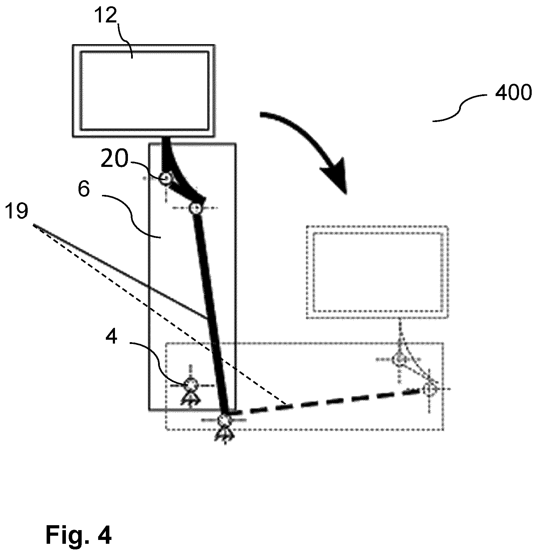

Die Schwenkbarkeit der verschiedenen Arme, die jeweils Aufbauten aufweisen können - wie einen Therapiebildschirm 12 am Schwenkarmgehäuse 6 des Laserschwenkarms 3 oder eine Untersuchungseinrichtung 15 am Untersuchungsschwenkarm 14 - führt bei einer unbeweglichen Befestigung dieser Aufbauten jeweils zu einer Verkippung dieser beim Schwenken der Arme aus einer Arbeitsposition heraus in eine Ruheposition. Gegebenenfalls sollen diese Aufbauten jedoch trotzdem nutzbar sein. Beispielsweise ist der Therapiebildschirm 12 auch in Ruheposition der Bildschirm, der einem Operateur in Arbeitsnähe zur Verfügung steht und beispielsweise von ihm für die Anzeige von Informationen oder für Eingaben genutzt werden soll. Deshalb sollte er sich auch in Ruheposition in unverkippter Stellung befinden. Ein ähnlicher Wunsch einer auch in Ruheposition unverkippten Stellung kann beispielsweise für ein Operationsmikroskop oder einer anderen Untersuchungseinrichtung mit Teilen, die bei einer Verkippung in unerwünschte Bewegung geraten würden, an einem Untersuchungsschwenkarm 14 bestehen. Dies ist über die Kopplung solcher Aufbauten mit dem jeweiligen Schwenkarm 3, 14 über eine Kopplungsmechanik 400 realisiert. Eine Ausgestaltung einer solchen Kopplungsmechanik 400 zeigt die Fig. 4.

Diese verschiedenen Lösungsbereiche, also das "Arm-in-Arm"-Prinzip 200, die Anordnung der Schwenkachsen 300 am Gerätekopf 1 eines ophthalmologischen Lasertherapiesystems 100 mit einem Laserschwenkarm 3 und mindestens einem Untersuchungsschwenkarm 14 sowie eine Kopplungsmechanik 400 für Aufbauten, die an Schwenkarmen unabhängig von der Position der Schwenkarme immer in unverkippter Stellung vorliegen sollen, können zusammen wie auch einzeln eingesetzt werden, um die Aufgabe der Erfindung zu lösen. Der größte Nutzen wird jedoch erzielt, wenn diese verschiedenen Lösungsbereiche gleichzeitig eingesetzt werden.

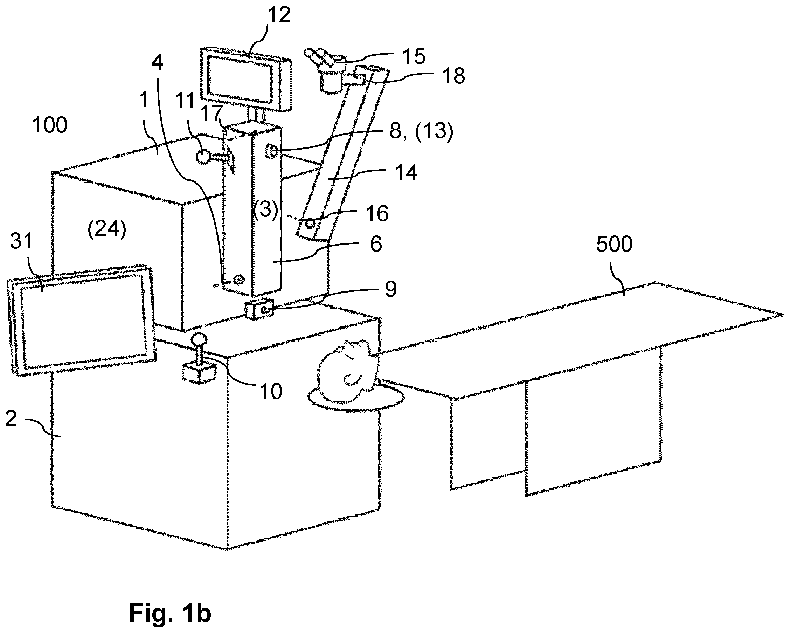

Die Fig. 1b bis 1d zeigen nun ein Ausführungsbeispiel eines erfindungsgemäßen ophthalmologischen Lasertherapiesystems 100, für das das "Arm-in-Arm"-Prinzip 200 durch einen Laserschwenkarm 3, der von einem Schwenkarmgehäuse 6 umfasst ist, realisiert ist, das ophthalmologische Lasertherapiesystem 100 einen zusätzlichen Untersuchungsschwenkarm 14 mit einem Operationsmikroskop 15 enthält, wobei die erste Achse 4 des Laserschwenkarm 3 und die zweite Achse 16 des Untersuchungsschwenkarms 14 am Gerätekopf 1 eine entsprechende Anordnung 300 zueinander aufweisen, und sowohl ein am Schwenkarmgehäuse 6 beweglich befestigter Therapiebildschirm 12 mit der Bewegung des Schwenkarmgehäuse 6 als auch ein Operationsmikroskop 15, das beweglich befestigt ist am Untersuchungsschwenkarm 14, mit der Bewegung des Untersuchungsschwenkarms 14 so gekoppelt sind, das der Therapiebildschirm 12 wie auch das Operationsmikroskop 15 stets unverkippt erhalten bleiben.

Ein ophthalmologisches Lasertherapiesystem 100 wie in diesem Ausführungsbeispiel gezeigt ist beispielsweise sehr gut einsetzbar für ein SMILE-Verfahren, aber auch für andere Verfahren zur Korrektur des Sehvermögens eines Auges oder für Katarakt-Operationen.

Dabei zeigt die Fig. 1b einen Standby-Modus dieses ophthalmologischen Lasertherapiesystems 100, in dem alle Schwenkarme 3, 6, 14 in einer Ruheposition, d.h. am Gerätekopf 1 nach oben geschwenkt platzsparend "geparkt" sind, und in der beispielsweise ein Patient auf der Patientenliege 500 entsprechend platziert und positioniert werden kann.

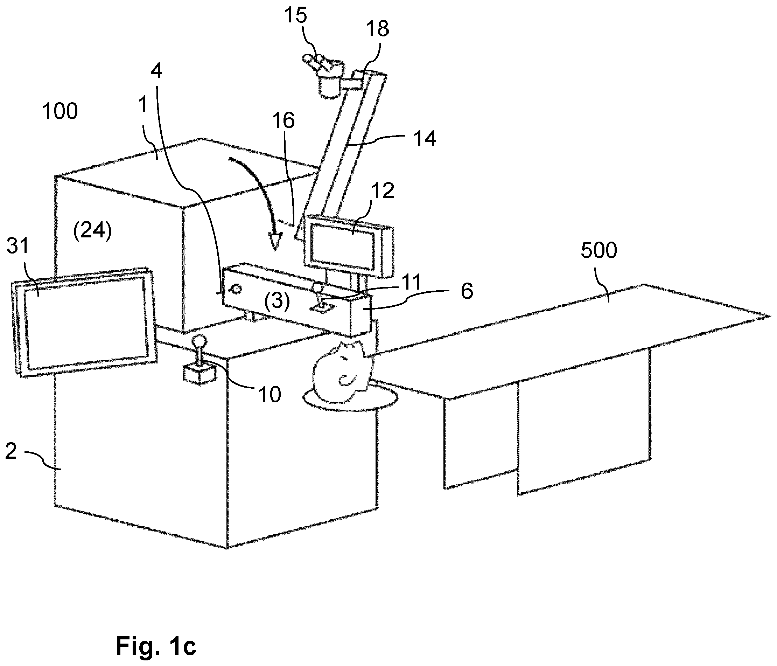

In der Fig. 1c ist hingegen ein Lasertherapiemodus dargestellt, also der Modus, in dem der Laserschwenkarm 3 in eine Arbeitsposition verbracht wurde. Der Untersuchungsschwenkarm 14 befindet sich hingegen nach wie vor in einer Ruheposition.

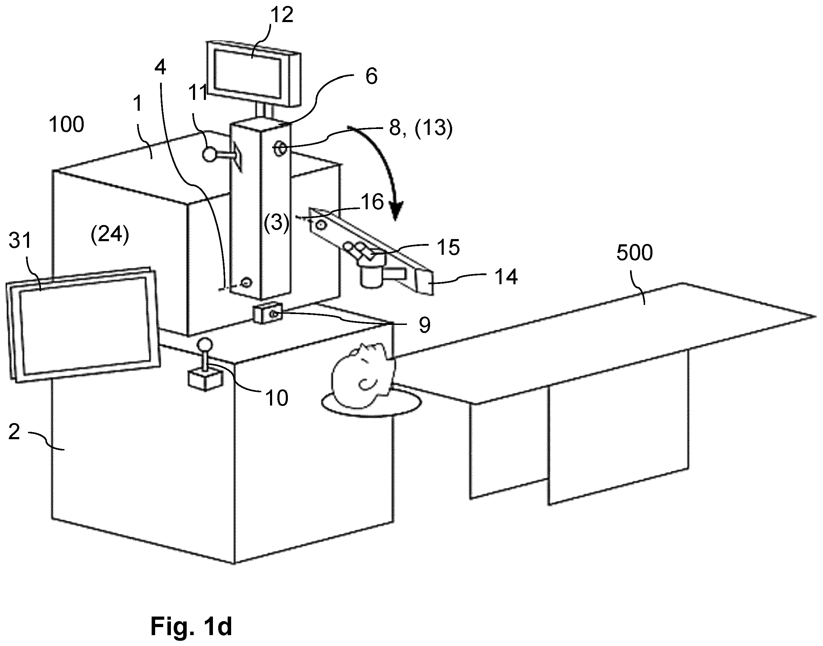

Die Fig. 1d zeigt schließlich einen Untersuchungsmodus des Ausführungsbeispiels des ophthalmologischen Lasertherapiesystems 100 unter Einsatz eines Operationsmikroskops 15. Dabei ist der Untersuchungsschwenkarm 14 in eine Arbeitsposition verbracht, während sich der Laserschwenkarm 3 und sein Schwenkarmgehäuse 6 in einer Ruheposition befinden.

Die Details sollen nun im Folgenden näher beschrieben werden.

Das Ausführungsbeispiel des ophthalmologischen Lasertherapiesystems 100 setzt sich zusammen aus einer Gerätebasis 2 und einem auf dieser Gerätebasis 2 in der Höhe über einer Bodenebene, also der z-Richtung, und in seiner Position in der Ebene, also in x- und y-Richtung, verstellbaren Gerätekopf 1. Der Gerätekopf 1 enthält einen ersten Teil der zur Durchführung der Lasertherapie erforderlichen Lasertherapieoptik. Der Gerätekopf 1 enthält im Ausführungsbeispiel auch die zur Erzeugung eines entsprechenden gepulsten Laserstrahls erforderliche Laserquelle, die hier eine Femtosekunden-Laserquelle ist.

Der zweite Teil der Lasertherapieoptik ist in einem Laserschwenkarm 3 um eine horizontale erste Achse 4 drehbar gelagert. Der Laserschwenkarm 3 kann um diese erste Achse 4 von einer Ruheposition, in der er ungefähr senkrecht nach oben ragt, in eine Arbeitsposition, in der er etwa waagerecht am Gerätekopf 1, also etwa parallel zur Bodenebene, angeordnet ist, sowie zurück geschwenkt werden.

Der Laserschwenkarm 3 mit seiner zweiten Lasertherapieoptik und der Laseraustrittsöffnung 8 ist von einem Gehäuse, dem Schwenkarmgehäuse 6 umgeben, derart dass das Schwenkarmgehäuse 6 eine Öffnung für die Laseraustrittsöffnung 8 lässt. Dieses Schwenkarmgehäuse 6 ist koaxial zum Laserschwenkarm 3 separat gelagert.

Die Fig. 2a und 2b stellen Details des "Arm-in-Arm" Prinzips 200 von Laserschwenkarm 3 und Schwenkarmgehäuse 6 in einem Ausführungsbeispiel des erfindungsgemäßen ophthalmologischen Lasertherapiesystems dar.

Das Schwenkarmgehäuse 6 schwenkt zunächst zusammen mit dem Laserschwenkarm 3 um einen Winkel von ca. 90° zwischen einer in etwa senkrechten Ruheposition bzw. Standby-Position und einer waagerechten Arbeitsposition. Die Bewegung ist durch Anschläge begrenzt.

Der Laserschwenkarm 3 kann insgesamt um einen größeren Winkel als das Schwenkarmgehäuse 6 bewegt werden. Damit kann die Laseraustrittsöffnung 8, an die ein Kontaktglas oder ein Patienteninterface zur Kopplung des Laserschwenkarms 3 mit dem zu behandelnden Auge des Patienten lösbar fixiert werden kann, mehr oder weniger weit aus dem Schwenkarmgehäuse 6 hervorstehend positioniert werden oder auch ganz in das Schwenkarmgehäuse 6 eingezogen werden.

In Ruheposition des Laserschwenkarms 3 sowie beim Schwenken des Laserschwenkarms 3 und seinem Schwenkarmgehäuse 6 von einer Ruheposition in eine Arbeitsposition wie auch von der Arbeitsposition in eine Ruheposition wird die Laseraustrittsöffnung 8 in das Schwenkarmgehäuse 6 eingezogen sein, wie dies in der Fig. 2a dargestellt ist. Damit ist der Laserschwenkarm 3 im Vergleich zu seinem Schwenkarmgehäuse 6 in einer leicht gekippten Stellung.

Ist das Schwenkarmgehäuse 6 in einer Arbeitsposition, also in der Waagerechten, angekommen, wird der Laserschwenkarm 3 nach unten freigegeben und leicht weiter geschwenkt, so dass auch er in eine annähernd waagerechte Position gelangt und die Laseraustrittsöffnung 8 aus dem Schwenkarmgehäuse 6 heraustritt. Der Laserschwenkarm 3 selbst ist dabei infolge seiner eigenen Gewichtsausgleichsvorrichtung 5 leichtgängig beweglich. In der annähernd waagerechten Arbeitsposition sowohl von Schwenkarmgehäuse 6 als auch von Laserschwenkarm 3 wie in der Fig. 2b dargestellt, also mit heraustretender Laseraustrittsöffnung 8 ist das ophthalmologische Lasertherapiesystem 100 im Lasertherapiemodus.

Eine sehr reibungsarme Gewichtsausgleichsvorrichtung 5 des Laserschwenkarms 3 ermöglicht eine Bewegung des Laserschwenkarms 3 mit geringer Kraft F, die für dieses Ausführungsbeispiel wesentlich kleiner als 3N ist, und damit eine gegen Quetschen sichere Kopplung mit einem zu behandelnden Auge eines Patienten: Die Kraft beträgt für dieses Beispiel 1,6 +/- 0,5N.

Das Schwenkarmgehäuse 6 ist mit einer eigenen Gewichtsausgleichsvorrichtung 7 versehen. Die separate koaxiale Lagerung von Laserschwenkarm 3 und dem diesen umgebenden Schwenkarmgehäuse 6 mit separaten Gewichtsausgleichsvorrichtungen 5, 7 und nach unten festem Anschlag 21 für das Schwenkarmgehäuse 6 vermeidet den entscheidenden Nachteil von entsprechenden Systemen nach dem Stand der Technik mit schwenkbaren Armen wie beispielsweise in US 8,771,262 B2 beschrieben. Dort ist nicht vorgesehen, dass die Optik in der Arbeitsposition des Laserschwenkarms mit einer für das Auge ungefährlichen Kraft und unabhängig von äußeren Einwirkungen, die ja in der erfindungsgemäßen Lösung durch das Schwenkarmgehäuse 6 abgehalten werden, nach oben auslenkbar ist.

Die Verwendung von separaten Gewichtsausgleichsvorrichtungen 5, 7 für das Schwenkarmgehäuse 6 und den Laserschwenkarm 3 hat mehrere Vorteile:
Eine motorgetriebene Bewegung bzw. Schwenkung des jeweiligen Arms 3, 6 um die erste Achse 4 kommt mit relativ leistungsschwachen Motoren aus. Diese müssen im Wesentlichen nur die Trägheit des Systems überwinden und können daher keine gefährlichen Kräfte am Schwenkarmgehäuse 6 aufbauen.

Zudem können der Laserschwenkarm 3 und das Schwenkarmgehäuse 6 - auch gemeinsam - jederzeit von Hand bewegt werden. Damit ist eine Befreiung des Patienten bei einem Defekt oder Stromausfall problemlos möglich.

Sollte die Patientenliege 500 ungewollt nach oben bewegt werden, so ist kein Quetschen des Patienten möglich, da der Laserschwenkarm 3 in seinem Schwenkarmgehäuse 6 mit einer ungefährlichen Kraft nach oben gedrückt werden kann.

Die Gewichtsausgleichsvorrichtung 7 des Schwenkarmgehäuses 6 enthält eine Gasfeder 26. Die Verwendung einer Gasfeder 26 hat folgende Vorteile: Sie erlaubt eine kompakte Bauweise der Gewichtsausgleichsvorrichtung 7 und kann eine Dämpfungsfunktion integrieren. Letzteres schützt vor zu hohen Geschwindigkeiten und dient als Abbremsung in den Endlagen der Schwenkbewegung.

Eine Gasfeder 26 hat aufgrund der Gasdichtungen eine hohe Reibung. Dies ist für den Laserschwenkarm 3 unerwünscht, aber beim Schwenkarmgehäuse 6 erwünscht. Sie bewirkt, dass das Schwenkarmgehäuse 6 in jeder Position passiv stehenbleibt. So kann z.B. das Schwenkarmgehäuse 6 auch motorisch in eine Art BereitschaftsStellung gebracht werden, wo es stromlos stehen bleibt und von dort per Hand vom Arzt in die Arbeitsposition gebracht wird. Das ist dann vorteilhaft, wenn der Arzt die manuelle Kontrolle über die Annäherung des Arms an den Patienten ausüben möchte.

Eine Gasfeder 26 wird also nur selten in der Gewichtsausgleichsvorrichtung 5 eines Laserschwenkarms 3 eingesetzt, während sie für die Gewichtsausgleichsvorrichtung 7 des Schwenkarmgehäuses 6 eine sehr bevorzugte Lösung darstellt.

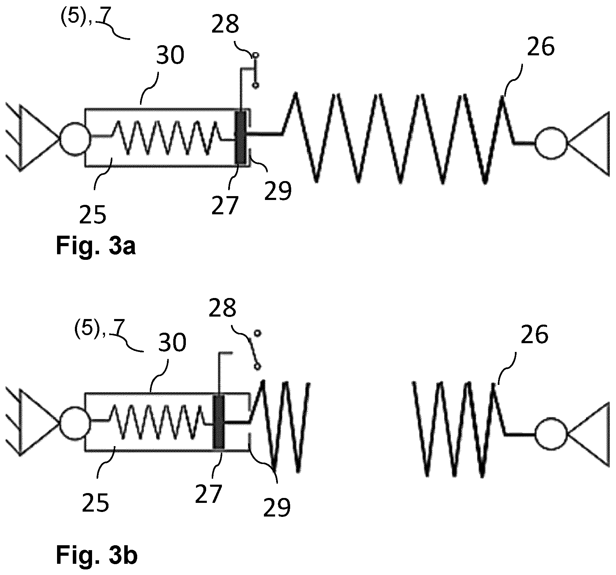

Die Fig. 3a und 3b zeigen eine Vorrichtung zur Überwachung der Funktion der Feder einer solchen bevorzugten Gewichtsausgleichsvorrichtung 7, mit der ein Versagen der Gasfeder 26 erkannt werden kann. Dabei stellt die Fig. 3a eine Situation dar, in der die Federkraft im gewünschten Bereich liegt, während die Fig. 3b die Situation zeigt, in der die Federkraft zu klein ist, weil beispielsweise, wie hier dargestellt, die Gasfeder 26 gebrochen ist. Im letzten Fall - also bei Problemen mit der Gasfeder 26 - ist dafür gesorgt, dass das Herunterschwenken des Schwenkarmgehäuses 6 verhindert wird, der Schwenkmechanismus also blockiert wird.

Die bevorzugte Gewichtsausgleichsvorrichtung 7 enthält zu diesem Zwecke eine Gasfeder 26, deren Spannkraft in ihrer Aufhängung 27 überwacht wird. Dies erfolgt dadurch, dass die Aufhängung 27 der Gasfeder 26 geringfügig beweglich und mit einem Schaltelement 28, das in diesem Fall ein Druckschalter ist, der durch einen entsprechenden Druck geschlossen gehalten wird, lösbar verbunden ist. Nur in diesem geschlossenen Zustand des Druckschalters wird der Schwenkmechanismus des Schwenkarmgehäuses 6 freigegeben. Hierzu dient eine elektromechanisch lösbare Verriegelung.

Ist die Federkraft der Gasfeder 26 nun ausreichend groß, dann wird eine Sensorfeder 25, die ebenfalls mit der Aufhängung 27 verbunden ist, in einer Führung 30 der Sensorfeder 25 bis zu einem Anschlag 29 gedehnt. Durch die Aufhängung 27 bzw. eine Verlängerung der Aufhängung 27 wird dann der Druckschalter 28 geschlossen gehalten, die Verriegelung gelöst, und das Schwenkarmgehäuse 6 ist schwenkbar um seine Schwenkachse, also um die erste Achse 4.

Ist die Federkraft der Gasfeder 26 jedoch zu klein, also kleiner als die Kraft der Sensorfeder 25, dann bewegt die Sensorfeder 25 die Aufhängung 27 der Gasfeder 26 in der Führung 30 zurück. Der Druckschalter 28 wird dabei geöffnet, die Verriegelung bleibt geschlossen und der Schwenkmechanismus wird nicht freigegeben.

Die Fig. 4 zeigt einen weiteren vorteilhaften Aspekt eines Ausführungsbeispiels des erfindungsgemäßen ophthalmologischen Lasertherapiesystems 100: Hier ist die Koppelmechanik des Therapiebildschirms 12 an die Bewegung des Schwenkarmgehäuses 6 als Beispiel einer an einen Schwenkarm gekoppelten Einrichtung 400 dargestellt.

Der Therapiebildschirm 12 ist am Schwenkarmgehäuse 6 beweglich befestigt. Der Therapiebildschirm 12 ist in diesem Fall auch gleichzeitig der Bildschirm eines Videomikroskops 13, welches den Blick aus der zweiten Lasertherapieoptik und der Laseraustrittsöffnung 8 heraus auf das zu behandelnde Auge zeigt. Das Videobild dieses Videomikroskops 13, das auf dem Therapiebildschirm 12 angezeigt wird, nutzt der Operateur beispielsweise zur Annäherung und zum Fixieren eines Kontaktglases oder eines anderweitigen Patienteninterfaces an das zu behandelnde Auge sowie zur Beobachtung der Ausführung der Laserschnitte.

Weitere für den Lasertherapieverlauf wichtige Informationen werden ebenfalls auf dem Therapiebildschirm 12 angezeigt. In diesem Ausführungsbeispiel ist der Therapiebildschirm 12 als Touchscreen ausgeführt und ermöglicht somit auch die Eingabe von Informationen oder die Navigation im Behandlungsablauf.

Der Therapiebildschirm 12 ist am Schwenkarmgehäuse 6 derart beweglich befestigt, dass er um eine zur horizontalen Schwenkachse, also der ersten Achse 4, des Schwenkarmgehäuse 6 parallele Achse 20 drehbar ist. Über eine Koppelstange 19 wird erreicht, dass der Therapiebildschirm 12 beim Schwenken des Laserschwenkarms 3 zusammen mit dem Schwenkarmgehäuse 6 immer in der gleichen Orientierung bleibt. Das ermöglicht eine Verwendung des Therapiebildschirms 12 sowohl in der Ruheposition als auch in der Arbeitsposition des Laserschwenkarms 3.

Wie weiter in den Fig. 1b bis 1d gezeigt, dient zur Vorpositionierung des Gerätekopfes 1 das Bild einer Kamera 9. Diese ist am Gerätekopf 1 befestigt und hat damit einen räumlich festen Bezug zur Position des Gerätekopfes 1. Die Position ist so gewählt, dass ein weitgehend parallaxefreier Blick auf das Arbeitsvolumen eines Therapielaserstrahls, insbesondere auf die mögliche Lage seines Fokus als Arbeitspunkt eines Therapieobjektives in der zweiten Lasertherapieoptik erfolgt.

Eine dem Bild der Kamera 9 überlagerte Grafik auf dem Therapiebildschirm 12 und/oder aber auf dem Planungsbildschirm 31 zeigt bereits im Standby-Modus, also in Ruheposition des Laserschwenkarms 3, die zu erwartende Position des Laserschwenkarmes 3 in seiner dann heruntergeschwenkten Arbeitsposition. Der Operateur kann mit Hilfe dieses Bildes den Gerätekopf 1 so vorpositionieren, dass sich der Laserschwenkarm 3 nach dem Herunterschwenken in seine Arbeitsposition, also im Laser-Modus, in einer bzgl. Grobpositionierung optimalen Position für den Behandlungsbeginn befindet, und nur noch eine Feinpositionierung in Bezug auf Strukturen des Auges nötig ist.

Am Schwenkarmgehäuse 6 ist weiterhin ein Joystick 11 zur Steuerung des Kopplungsvorganges an den Patienten angebracht. Joystick 11, Laseraustrittsöffnung 8 der Lasertherapieoptik und Videobild des Auges sind in der Arbeitsposition an einer Vertikalen 23 ausgerichtet, um eine für Rechts- und Linkshänder gleichermaßen ergonomische Bedienung zu ermöglichen.

Außerdem enthält dieses Ausführungsbeispiel des erfindungsgemäßen ophthalmologischen Lasertherapiesystems 100 einen Kraftsensor, der zur sicherheitstechnisch wichtigen, automatischen Lageregelung beiträgt: Der Kraftsensor ist hierfür so angeordnet, dass die zweite Lasertherapieoptik auf dem Kraftsensor liegt, wenn sich der Laserschwenkarm in Arbeitsposition befindet.

Ein Druck des Patientenauges gegen die zweite Lasertherapieoptik bzw. die Laseraustrittsöffnung entlastet den Kraftsensor. Dies führt zu einer automatischen Bewegung der Position des Gerätekopfes 1 derart, dass sich der Laserschwenkarm 3 in Arbeitsposition vom Patienten entfernt. Ein höherer Druck auf den Kraftsensor entsteht durch einen Zug des Patienten: Hierdurch wird der Gerätekopf 1 dann in die entgegengesetzte Richtung bewegt.

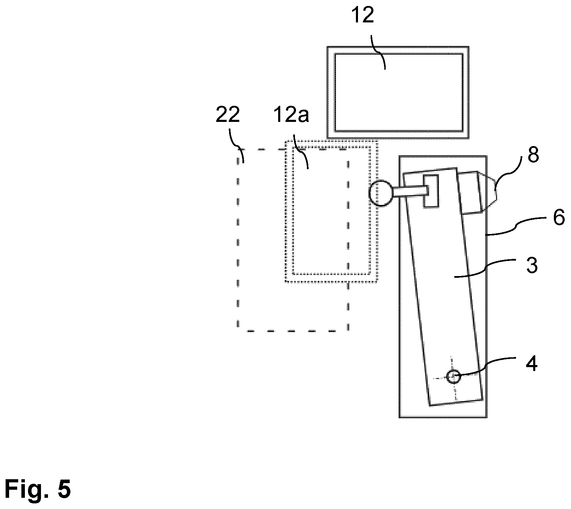

Die Fig. 5 zeigt nun den Gewinn an Arbeitsraum infolge des unverkippt bleibenden Therapiebildschirmes 12 in Ruheposition: Der Raum 22 kann für Komponenten der Lasereinrichtung 24 im Gerätekopf 1 genutzt werden. Wäre der Therapiebildschirm 12 fest am Schwenkarmgehäuse 6 befestigt, wie als Referenzzeichen 12a dargestellt, so wäre der Raum 22 für den in Ruheposition dorthin schwenkenden Therapiebildschirm 12 zuzüglich eines Sicherheitsabstandes blockiert.

Im Folgenden wird nun ein typischer Behandlungsablauf, wie er beispielsweise für eine SMILE-Behandlung bzw. als Teil einer SMILE-Behandlung eingesetzt werden kann, unter Nutzung eines oben beschriebenen ophthalmologischen Lasertherapiesystems 100 beschrieben:
Zunächst erfolgt die Planung der Behandlungs- bzw. Therapieparameter an einem Planungsbildschirm 31, der in diesem Ausführungsbeispiel ebenfalls direkt am ophthalmologischen Lasertherapiesystem 100 angeordnet ist. Alternativ kann der Planungsbildschirm 31 aber auch vom ophthalmologischen Lasertherapiesystem 100 räumlich getrennt sein. Während der Planung befindet sich das ophthalmologische Lasertherapiesystem 100 vorzugsweise im einer Standby-Position, d.h., der Laserschwenkarm 3 wie ggf. auch der Untersuchungsschwenkarm 14 ist in Ruheposition am System vertikal hochgeschwenkt.

Der Patient wird auf der Patientenliege 500 platziert. Aufgrund des hochgeschwenkten Laserschwenkarms 3 ist dies bequem möglich.

Der Operateur positioniert dann die Höhe des Gerätekopfes 1 mittels eines Joysticks 10 an diesem Gerätekopf 1, mit dem die Translationsbewegung des Gerätekopfes 1 über der Gerätebasis 2 gesteuert werden kann. Dabei orientiert er sich an dem von der Kamera 9 gelieferten Bild, welches auf dem Therapiebildschirm 12 und/oder auf dem Planungsbildschirm 31 inklusive eines überlagerten Sinnbildes eines heruntergeschwenkten Laserschwenkarmes 3 sichtbar ist. Alternativ zum Joystick kann in anderen Ausführungen die Positionierung auch durch Eingaben an einem der beiden Bildschirme 12, 31 oder über Tasten am Lasertherapiesystem 100 geschehen.

Der Operateur löst das motorische Herunterschwenken des Laserschwenkarms 3 in und gemeinsam mit seinem Schwenkarmgehäuse 6 aus; ein entsprechender hierfür verwendeter Taster ist in den Figuren nicht dargestellt. Durch die Vorpositionierung und die noch eingezogene Laseraustrittsöffnung 8 des Laserschwenkarms verbleibt ein Freiraum zwischen der Laseraustrittsöffnung 8 und dem Patientenauge, der günstig zwischen 50mm und 150mm groß ist.

Falls dies noch nicht in der Ruheposition des Laserschwenkarms 3 geschehen ist, wird nun ein Kontaktglas an die Laseraustrittsöffnung 8 angesetzt. Das Festhalten des Kontaktglases an der Laseraustrittsöffnung 8 erfolgt mittels eines Unterdrucks. Das Ein- und Ausschalten des Festhaltens mittels Unterdruck geschieht dabei, indem das Kontaktglas gegen die Laseraustrittsöffnung 8 gedrückt wird, diese dabei in ihrer eingezogenen Stellung noch geringfügig bewegt wird und den Schaltvorgang auslöst. Dies ist vorteilhaft gegenüber bislang üblichen Lasertherapiesystemen: Dort wird das Festhalten des Kontaktglases separat geschaltet. Somit passiert es, dass das Kontaktglas beim Lösen herunterfällt. In der hier beschriebenen Lösung hat der Operateur bzw. Bediener das Kontaktglas beim Schaltvorgang hingegen immer in der Hand.

Der Operateur veranlasst dann die Freigabe der Bewegung des Laserschwenkarms 3 innerhalb des Schwenkarmgehäuses 6 mittels einer Joystickdrehung des Joysticks 11 am Schwenkarmgehäuse 6, oder alternativ mittels eines nicht dargestellten separaten Tasters. In anderen Ausführungen ist auch ein automatisches Auslösen der Bewegung durch das angesetzte Kontaktglas möglich. Die Laseraustrittsöffnung 8 mit dem Kontaktglas bewegt sich dabei mit der durch die Gewichtsausgleichsvorrichtung 5 des Laserschwenkarms 3 eingestellten Kraft, die kleiner als 3N am Kontaktglas bzw. in Höhe der Laseraustrittsöffnung 8 ist, auf das Auge zu. Der Bewegungsweg beträgt dabei ungefähr 50mm, ein ganz allgemein für alle erfindungsgemäßen ophthalmologischen Lasertherapiesysteme 100 sinnvoller Bereich für diesen Bewegungsweg ist 30mm bis 100mm. Damit verbleibt immer noch ein Sicherheitsabstand zum Auge, der ungefähr 30mm beträgt, bzw. ganz allgemein sinnvoll einen Wert zwischen 10mm und 100mm annimmt.

Schließlich erfolgt die Andockphase, also die Phase, in der das Kontaktglas fixiert wird: Der Operateur steuert dabei mit dem Joystick 11 unter Beobachtung mittels des Videomikroskops 13 das Kontaktglas auf das Auge des Patienten. Bei Erreichen der richtigen Position wird die Fixierung des Auges durch ein Ansaugen des Auges am Kontaktglas mit einem Taster am Joystick 11 veranlasst. In einer Ausgestaltung ist es möglich, die richtige Positionierung bzw. Zentrierung des Kontaktglases oder eines anderen Patienteninterfaces auf dem Auge unterstützen, indem das Videomikroskop-Bild verarbeitet und zur Steuerung des Gerätekopfes 1 herangezogen wird.

Damit kann nun schließlich der eigentliche Lasertherapieschritt durch Einschalten des Laserstrahls, der durch die Lasertherapieoptiken und die Laseraustrittsöffnung geleitet und im Patientenauge fokussiert wird, mittels eines Fußschalters, der hier nicht dargestellt wird, gestartet werden.

Nach Abschluss dieses Lasertherapieschritts wird das Ansaugen des Auges mittels Unterdruck gelöst, indem hier wieder der Druck erhöht wird, der Laserschwenkarm 3, und damit auch die Laseraustrittsöffnung 8, wieder in das Schwenkarmgehäuse 6 eingeschwenkt und der Gerätekopf 1 durch ein Verfahren in z-Richtung etwas hochgefahren. Damit ist wieder ein Sicherheitsabstand zum Auge vorhanden. Aus dieser Position heraus könnte nun gegebenenfalls erneut angedockt werden.
In der Regel ist dies jedoch nicht nötig. Das Kontaktglas oder das Patienteninterface kann von der Laseraustrittsöffnung 8 abgenommen werden, wobei die Freigabe durch ein kurzes Drücken nach oben erfolgt.

Der Laserschwenkarm 3 wird nun zusammen mit seinem Schwenkarmgehäuse 6 wieder hochgeschwenkt, der Freiraum über dem Patienten ist wieder hergestellt. Es können nun weitere Arbeitsschritte durchgeführt werden, oder der Patient kann seine Position auf der Patientenliege 500 verlassen. Das Hochschwenken des Laserschwenkarms 3 mit seinem Schwenkarmgehäuse 6 wird elektronisch, hier durch einen Tastendruck, initiiert. Alternativ kann der Laserschwenkarms 3 mit seinem Schwenkarmgehäuse 6 manuell angeschoben werden, ein Positionssensor am Schwenkarmgehäuse erkennt dies, daraufhin übernimmt ein Motor die Bewegung.

Sollen jedoch beide Augen eines Patienten behandelt werden, so kann vor dem Hochschwenken des Laserschwenkarms 3 mit seinem Schwenkarmgehäuse 6 in seine Ruheposition der Gerätekopf 1 durch eine Translationsbewegung in x- und/oder y-Richtung über der Gerätebasis 2 so bewegt werden, dass der Laserschwenkarm 3 mit seinem Schwenkarmgehäuse 6 über dem anderen Auge positioniert wird. Eine Behandlung des zweiten Auges kann dann in gleicher Weise erfolgen, indem ein neues Kontaktglas oder Patienteninterface an der Laseraustrittsöffnung 8 und mittels Unterdruck festgehalten wird, und alle daraufhin folgenden Schritte wie oben beschrieben ausgeführt werden.

Am Gerätekopf 1 ist in diesem Ausführungsbeispiel eines erfindungsgemäßen ophthalmologischen Lasertherapiesystems 100 weiterhin auch ein Untersuchungsschwenkarm 14 um eine zweite Achse 16 schwenkbar befestigt, der eine Untersuchungseinrichtung, in diesem Fall ein Operationsmikroskop 15, enthält. Ein solches Operationsmikroskop ist beispielsweise für den zweiten Hauptarbeitsschritt der "SMILE"-Behandlung erforderlich oder zumindest angeraten. Im vorliegenden Ausführungsbeispiel enthält das Operationsmikroskop 15 neben der notwendigen Beleuchtung eine Kamera zur Videoaufzeichnung und einen Spaltprojektor für erweiterte Beobachtungsmöglichkeiten. Kamera sowie Spaltprojektor können jedoch an dieser Stelle auch entweder ganz fehlen oder einzeln eingesetzt werden.

Die Schwenkachse des Untersuchungsschwenkarms 14, also die zweite Achse 16, ist in einer besonders günstigen Lage im Raum positioniert. Diese erlaubt es, das Operationsmikroskop 15 am Untersuchungsschwenkarm 14 mit nur einer Schwenkbewegung aus seiner Ruheposition, in der der Untersuchungsschwenkarm 14 ebenfalls- entweder in einer ebenfalls senkrechten Position oder in einer schrägen Position - hochgeschwenkt ist, in seine Arbeitsposition zu bringen.

Auch diese Arbeitsposition wird durch eine Begrenzung der Drehbewegung des Untersuchungsschwenkarms 14 mittels eines Anschlages definiert. Sie hat dabei die besondere Eigenschaft, mit der Arbeitsposition des Laserschwenkarms 3 mit seiner zweiten Lasertherapieoptik und seiner Laseraustrittsöffnung 8 zusammenzufallen und damit erfindungsgemäß eine Positionsänderung des Patienten während der Behandlung zu vermeiden.

Die Ruheposition des Untersuchungsschwenkarms 14 mit seinem Operationsmikroskop 15 ist günstig so gewählt, dass sich das Operationsmikroskop 15 dann hinter dem Therapiebildschirm 12 befindet. Es gibt damit einerseits den Raum zum Ein- und Ausstieg des Patienten frei und ermöglicht andererseits ein kompaktes ophthalmologischen Lasertherapiesystem 100 im Standby-Modus.

Im hier beschriebenen Ausführungsbeispiel ist die zweite Achse 16, also die Schwenkachse des Untersuchungsschwenkarms 14, zur ersten Achse 4, die die Schwenkachse des Laserschwenkarms 3 und seines Schwenkarmgehäuses 6 ist, besonders vorteilhaft angeordnet, um das ophthalmologische Lasertherapiesystem 100 möglichst kompakt ausführen zu können:
Die zweiten Achse 16 des Untersuchungsschwenkarms 14 bildet einen Winkel zu einer senkrechten Ebene durch die erste Achse 4 des Laserschwenkarmes 3 von 0° und einen Winkel zu einer waagerechten Ebene durch die erste Achse 4 des Laserschwenkarms 3 von 30°.

Die zweite Achse 16 verläuft durch einen Punkt, der sich 320mm oberhalb der Drehachse des Laserschwenkarmes 3, 320mm hinter der Arbeitsposition des Laseraustrittsöffnung 8 und 50mm von der senkrechten Ebene durch die Drehachse des Laserschwenkarmes 3 entfernt befindet.

Das Operationsmikroskop 15 ist am Untersuchungsschwenkarm 14 ähnlich gelagert wie der Therapiebildschirm 12 am Schwenkarmgehäuse 6: drehbar um eine Achse 18, die parallel ist zur Drehachse des Untersuchungsschwenkarms 14, also der zweiten Achse 16, und mit einer Koppelstange 19 mit dem Gerätekopf 1 drehbar verbunden. Die Koppelstange 19 hat dabei eine zur ersten Achse 4 parallele Drehachse am Gerätekopf 1.

Der Winkel der ersten Achse 4 zur zweiten Achse 16 im Raum muss dabei in der Halterung des Operationsmikroskops 15, also der Lage der Achse 18, kompensiert werden, damit das Operationsmikroskop 15 wieder unverkippt im Raum steht.

Damit bleibt das Operationsmikroskop 15 in jeder Stellung des Untersuchungsschwenkarms 14 in seiner Arbeitslage. Das hat verschiedene Vorteile: Bewegliche Anbauteile am Operationsmikroskop 15, wie z.B. ein Spaltprojektor, verrutschen nicht. Zudem ist das ästhetische Erscheinungsbild einheitlich.

Ähnlich wie das Schwenkarmgehäuse 6 des Laserschwenkarms 3 ist auch der Untersuchungsschwenkarm 14 mit einer Gewichtsausgleichvorrichtung 7 versehen, die eine Gasfeder 25 enthält. Das hat auch hier wieder die bereits oben beschriebenen Vorteile: Der Einsatz eines damit möglichen schwachen Motors zur Bewegung des Untersuchungsschwenkarms 14 bietet Sicherheit vor einer Quetschgefahr des Patientenkopfes. Die entsprechende Ausführung des Antriebs kann relativ klein gehalten werden und ist kostengünstiger als eine Lösung, die das komplette Gewicht ausgleichen muss. Zudem ist auch der Untersuchungsschwenkarm 14 jederzeit von Hand beweglich.

Beim einem Untersuchungsschwenkarm 14, der ein Operationsmikroskop 15 enthält - oder aber andere Aufbauten, für die kein Kontakt mit dem Auge vorgesehen ist, die also keine hervorstehenden Optiken o.ä. enthalten, ist hier die Quetschgefahr des Patientenkopfes wesentlich, und es kann mit den maximalen Kräften bzw. kleineren Kräften auf den Patientenkopf von kleiner 65N - wie oben beschrieben - gearbeitet werden. In anderen Ausführungen jedoch, bei denen eine Untersuchungseinrichtung 15 einen Kontakt mit dem Auge verlangt, muss wieder das "Arm-in-Arm"-Prinzip angewendet werden und folglich für den dann inneren Schwenkarm, der die das Auge kontaktierende Untersuchungseinrichtung 15 enthält, eine Quetschgefahr des Auges vermieden werden, indem mit wesentlich kleineres maximalen Kräften von kleiner 3N auf das Auge - wie oben beschrieben - gearbeitet wird.

Bei Vorhandensein eines solchen Untersuchungsschwenkarms 14 mit einem Operationsmikroskop 15 kann die vollständige SMILE-Behandlung mit dem erfindungsgemäßen ophthalmologischen Lasertherapiesystem 100 durchgeführt werden. Dazu wird die Behandlung nach dem Hochschwenken des Laserschwenkarms 3 mit seinem Schwenkarmgehäuse 6 in seine Ruheposition nach der Beendigung des eigentlichen Lasertherapieschritts wie hier beschrieben, folgendermaßen fortgesetzt:
Der Operateur initiiert das motorische Herunterschwenken des Untersuchungsschwenkarms 14 durch Tastendruck. Der Motor bewegt den Untersuchungsschwenkarm 14 in seine Arbeitsposition, wo er auf einem Anschlag aufliegt. Die Arbeitsposition ist durch die günstige Wahl der Lage der beiden Schwenkachsen, also der ersten Achse 4 und der zweiten Achse 16 und die durch den Anschlag bestimmte Endposition des Untersuchungsschwenkarms 14 so bestimmt, dass das weiter zu behandelnde Auge direkt nach dem Herunterschwenken des Untersuchungsschwenkarmes 14 im Untersuchungsvolumen des Operationsmikroskops 15 liegt.

Geringfügige Korrekturen, sofern nötig, sind durch die Verstellung der Position des Gerätekopfes 1 zur Gerätebasis 2 mittels Translationsbewegungen möglich. Hierzu dient entweder der an der Gerätebasis 2 vorhandene Joystick 10, ein separates Fußschaltpult oder ein am Operationsmikroskop 15 vorhandener Joystick.

Ist der Untersuchungsschwenkarm 14 mit dem Operationsmikroskop 15 entsprechend positioniert, wird durch den Operateur die Lentikelextraktion durchgeführt.

Nach Beendigung der Lentikelextraktion wird der Untersuchungsschwenkarm 14 mit dem Operationsmikroskop 15 motorisch hochgeschwenkt und damit in seine Ruheposition zurückgeschwenkt. Dies kann mittels eines Tastendrucks oder aber - wie oben schon beschrieben für das Schwenkarmgehäuse 6 und den Laserschwenkarm 3 - durch ein Anschieben initiiert werden. Der Freiraum über dem Patienten ist damit wieder hergestellt.

Die vorstehend genannten und in verschiedenen Ausführungsbeispielen erläuterten Merkmale der Erfindung sind dabei nicht nur in den beispielhaft angegebenen Kombinationen, sondern auch in anderen Kombinationen oder allein einsetzbar, ohne den Rahmen der vorliegenden Erfindung zu verlassen.

Hier beschriebene Verfahrensmerkmale können dabei funktionelle Merkmale der beschriebenen Vorrichtung darstellen.

## Patentansprüche

1. Ophthalmologisches Lasertherapiesystem (100) umfassend:
- eine Gerätebasis (2),
- einen Gerätekopf (1),
- eine Lasereinrichtung (24), die eine Laserquelle, eine erste Lasertherapieoptik und einen Laserschwenkarm (3) mit einer zweiten Lasertherapieoptik und einer Laseraustrittsöffnung (8) enthält,
wobei
- der Gerätekopf (1) auf der Gerätebasis (2) mittels einer Translationsbewegung in einer x-y-Ebene verfahrbar ist und
- der Laserschwenkarm (3) am Gerätekopf (2) um eine erste Achse (4), bevorzugt eine horizontale erste Achse, schwenkbar befestigt ist,
- ein Untersuchungsschwenkarm (14) mit einer Untersuchungseinrichtung (15), die ein Untersuchungsvolumen definiert, am Gerätekopf (1) um eine zweite Achse (16) schwenkbar befestigt ist,
- **dadurch gekennzeichnet, dass** sowohl der Laserschwenkarm (3) als auch der Untersuchungsschwenkarm (14) schwenkbar ist zwischen einer Ruheposition und einer Arbeitsposition,
- und beide Achsen (4, 16) in ihrer Lage so zueinander angeordnet sind, dass ein Arbeitsvolumen eines Laserstrahls, wenn sich der Laserschwenkarm (3) in der Arbeitsposition befindet, ein Teilvolumen des Untersuchungsvolumens der Untersuchungseinrichtung (15) am Untersuchungsschwenkarm (14) ist, wenn sich dieser in der Arbeitsposition befindet, während der Laserschwenkarm (3) und der Untersuchungsschwenkarm (15) in Ruheposition nicht im Schwenkbereich des jeweils anderen Schwenkarmes (3, 15) angeordnet sind,
- wobei die zweite Achse (16) des Untersuchungsschwenkarms (14) nichtparallel zur ersten Achse (4) des Laserschwenkarms (3) verläuft.

2. Ophthalmologisches Lasertherapiesystem (100) nach Anspruch 1, **dadurch gekennzeichnet, dass** der Laserschwenkarm (3) von einem Schwenkarmgehäuse (6) umfasst ist, das koaxial zum Laserschwenkarm (3) am Gerätekopf (1) separat schwenkbar befestigt ist.

3. Ophthalmologisches Lasertherapiesystem (100) nach Anspruch 2, **dadurch gekennzeichnet, dass** der Laserschwenkarm (3) um einen größeren Winkel als das Schwenkarmgehäuse (6) schwenkbar ist.

4. Ophthalmologisches Lasertherapiesystem (100) nach Anspruch 2 oder 3, **dadurch gekennzeichnet, dass** der Laserschwenkarm (3) sowie das Schwenkarmgehäuse (6) jeweils eine separate Gewichtsausgleichsvorrichtung (5, 7) aufweisen.

5. Ophthalmologisches Lasertherapiesystem (100) nach einem der Ansprüche 1 bis 4, **dadurch gekennzeichnet, dass** der Gerätekopf (1) auf der Gerätebasis (2) auch in z-Richtung verfahrbar ist.

6. Ophthalmologisches Lasertherapiesystem (100) nach einem der Ansprüche 1 bis 5, **dadurch gekennzeichnet, dass** es eine Vorpositionierungseinrichtung (9) zur Vorpositionierung des Gerätekopfes (1) umfasst, die vorzugsweise eine Kamera enthält.

7. Ophthalmologisches Lasertherapiesystem (100) nach einem der Ansprüche 2 bis 6, **dadurch gekennzeichnet, dass** der Laserschwenkarm (3) und/oder das Schwenkarmgehäuse (6) mindestens eine Detektionseinrichtung (13), insbesondere eine Videomikroskop-Vorrichtung oder eine OCT-Vorrichtung, enthält.

8. Ophthalmologisches Lasertherapiesystem (100) nach einem der Ansprüche 2 bis 7, **dadurch gekennzeichnet, dass** eine Eingabe- und/oder Ausgabevorrichtung (12), insbesondere ein Therapiebildschirm, am Schwenkarmgehäuse (6) beweglich befestigt ist.

9. Ophthalmologisches Lasertherapiesystem (100) nach einem der Ansprüche 1 bis 8, wobei die Untersuchungseinrichtung (15) ein Operationsmikroskop ist.

10. Ophthalmologisches Lasertherapiesystem (100) nach Anspruch 8 oder 9, **dadurch gekennzeichnet, dass** die Eingabe- und/oder Ausgabevorrichtung (12) um eine zur ersten Achse (4) des Schwenkarmgehäuses (6) und/oder die Untersuchungseinrichtung (15) um eine zur zweiten Achse (16) des Untersuchungsschwenkarms (14) parallele Achse (17, 18) drehbar, und über eine Koppelvorrichtung (19) an die Bewegung des Schwenkarmgehäuses (6) oder des Untersuchungsschwenkarms (14) gekoppelt ist.

11. Ophthalmologisches Lasertherapiesystem (100) nach einem der Ansprüche 1 bis 10, **dadurch gekennzeichnet, dass** die erste und/oder die zweite Lasertherapieoptik ein Scansystem mit mindestens zwei Scannern enthält, und die erste Achse (4) des Laserschwenkarms (3) in der Strahlachse zwischen den Scannern angeordnet ist.

12. Ophthalmologisches Lasertherapiesystem (100) nach einem der Ansprüche 1 bis 11, **gekennzeichnet durch** eine Lageregelungsvorrichtung zur Regelung der Lage zwischen dem Laserschwenkarm (3) und /oder dem Untersuchungsschwenkarm (14) in Arbeitsposition und einem Patientenauge, die eingerichtet ist, die Lage des Gerätekopfes (1) der Position eines Patientenauges nachzuführen.

## Claims

1. Ophthalmological laser therapy system (100) comprising:
- an equipment base (2),
- an equipment head (1),
- a laser device (24) that contains a laser source, a first laser therapy optical unit and a laser pivot arm (3) with a second laser therapy optical unit and a laser exit aperture (8),
wherein
- the equipment head (1) is displaceable on the equipment base (2) in an xy-plane by means of a translational movement and
- the laser pivot arm (3) is fastened to the equipment head (1) in a manner pivotable about a first axis (4), preferably a horizontal first axis,
- an examination pivot arm (14) with an examination device (15), which defines an examination volume, is fastened to the equipment head (1) in a manner pivotable about a second axis (16),
- **characterized in that** both the laser pivot arm (3) and the examination pivot arm (14) are pivotable between a rest position and a work position,
- and both axes (4, 16) are arranged relative to one another in terms of their relative position such that a work volume of a laser beam, when the laser pivot arm (3) is in the work position, is a partial volume of the examination volume of the examination device (15) on the examination pivot arm (14), when the latter is in the work position, while the laser pivot arm (3) and the examination pivot arm (15) are not arranged in the pivot region of the respective other pivot arm (3, 15) in their respective rest position,
- wherein the second axis (16) of the examination pivot arm (14) extends in a non-parallel fashion relative to the first axis (4) of the laser pivot arm (3).

2. Ophthalmological laser therapy system (100) according to Claim 1, **characterized in that** the laser pivot arm (3) is encompassed by a pivot arm housing (6), which is fastened in a separately pivotable manner on the equipment head (1) in coaxial fashion relative to the laser pivot arm (3).

3. Ophthalmological laser therapy system (100) according to Claim 2, **characterized in that** the laser pivot arm (3) is pivotable by a greater angle than the pivot arm housing (6).

4. Ophthalmological laser therapy system (100) according to Claim 2 or 3, **characterized in that** the laser pivot arm (3) and the pivot arm housing (6) each have a separate weight balancing apparatus (5, 7).

5. Ophthalmological laser therapy system (100) according to any of Claims 1 to 4, **characterized in that** the equipment head (1) is also displaceable in the z-direction on the equipment base (2).

6. Ophthalmological laser therapy system (100) according to any of Claims 1 to 5, **characterized in that** it comprises a pre-positioning device (9) for pre-positioning of the equipment head (1), said pre-positioning device preferably containing a camera.

7. Ophthalmological laser therapy system (100) according to any of Claims 2 to 6, **characterized in that** the laser pivot arm (3) and/or the pivot arm housing (6) contains at least one detection device (13), in particular a video microscope apparatus or an OCT apparatus.

8. Ophthalmological laser therapy system (100) according to any of Claims 2 to 7, **characterized in that** an input and/or output apparatus (12), in particular a therapy screen, is movably fastened to the pivot arm housing (6).

9. Ophthalmological laser therapy system (100) according to any of Claims 1 to 8, wherein the examination device (15) is a surgical microscope.

10. Ophthalmological laser therapy system (100) according to Claim 8 or 9, **characterized in that** the input and/or output apparatus (12) is rotatable about an axis (17) that is parallel to the first axis (4) of the pivot arm housing (6) and/or the examination device (15) is rotatable about an axis (18) that is parallel to the second axis (16) of the examination pivot arm (14), and is coupled by way of a coupling apparatus (19) to the movement of the pivot arm housing (6) or of the examination pivot arm (14).

11. Ophthalmological laser therapy system (100) according to any of Claims 1 to 10, **characterized in that** the first and/or the second laser therapy optical unit contains a scan system with at least two scanners, and the first axis (4) of the laser pivot arm (3) is arranged in the beam axis between the scanners.

12. Ophthalmological laser therapy system (100) according to any of Claims 1 to 11, **characterized by** a position regulating apparatus for regulating the relative position between the laser pivot arm (3) and/or the examination pivot arm (14) in a work position and a patient's eye, said position regulating apparatus being configured to reposition the relative position of the equipment head (1) according to the position of a patient's eye.

## Revendications

1. Système de thérapie ophtalmique au laser (100) comprenant :
- une base d'appareil (2),
- une tête d'appareil (1),
- un dispositif à laser (24), qui contient une source laser, une première optique de thérapie au laser et un bras pivotant de laser (3) avec une deuxième optique de thérapie au laser et une ouverture de sortie laser (8),
- la tête d'appareil (1) pouvant être déplacée sur la base d'appareil (2) au moyen d'un mouvement de translation dans un plan x-y et
- le bras pivotant de laser (3) étant fixé à la tête d'appareil (1) de manière à pouvoir pivoter autour d'un premier axe (4), de préférence un premier axe horizontal,
- un bras pivotant d'examen (14) pourvu d'un dispositif d'examen (15), qui définit un volume d'examen, étant fixé à la tête d'appareil (1) de manière à pouvoir pivoter autour d'un deuxième axe (16),
- **caractérisé en ce qu'**à la fois le bras pivotant de laser (3) ainsi que le bras pivotant d'examen (14) peuvent pivoter entre une position de repos et une position de travail,
- et les deux axes (4, 16) sont positionnés l'un par rapport à l'autre de telle sorte que, lorsque le bras pivotant de laser (3) se trouve dans la position de travail, un volume de travail d'un faisceau laser est un volume partiel du volume d'examen du dispositif d'examen (15) au niveau du bras pivotant d'examen (14) lorsque celui-ci se trouve dans la position de travail, tandis que le bras pivotant de laser (3) et le bras pivotant d'examen (15) ne sont pas disposés dans la zone de pivotement de l'autre bras pivotant (3, 15) respectif lorsqu'ils se trouvent en position de repos,
- le deuxième axe (16) du bras pivotant d'examen (14) ne suivant pas un tracé parallèle au premier axe (4) du bras pivotant de laser (3).

2. Système de thérapie ophtalmique au laser (100) selon la revendication 1, **caractérisé en ce que** le bras pivotant de laser (3) est entouré par un boîtier de bras pivotant (6), lequel est fixé à la tête d'appareil (1) de manière coaxiale au bras pivotant du laser (3) de manière à pouvoir pivoter séparément.

3. Système de thérapie ophtalmique au laser (100) selon la revendication 2, **caractérisé en ce que** le bras pivotant de laser (3) peut pivoter sur un angle plus grand que le boîtier de bras pivotant (6).

4. Système de thérapie ophtalmique au laser (100) selon la revendication 2 ou 3, **caractérisé en ce que** le bras pivotant de laser (3) ainsi que le boîtier de bras pivotant (6) possèdent chacun un dispositif de compensation de poids (5, 7) séparé.

5. Système de thérapie ophtalmique au laser (100) selon l'une des revendications 1 à 4, **caractérisé en ce que** la tête d'appareil (1) peut également être déplacée dans la direction z sur la base d'appareil (2).

6. Système de thérapie ophtalmique au laser (100) selon l'une des revendications 1 à 5, **caractérisé en ce qu'**il comprend un dispositif de pré-positionnement (9) destiné à pré-positionner la tête d'appareil (1), qui contient de préférence une caméra.

7. Système de thérapie ophtalmique au laser (100) selon l'une des revendications 2 à 6, **caractérisé en ce que** le bras pivotant de laser (3) et/ou le boîtier de bras pivotant (6) contient au moins un dispositif de détection (13), notamment un arrangement de vidéo-microscopie ou un arrangement d'OCT.

8. Système de thérapie ophtalmique au laser (100) selon l'une des revendications 2 à 7, **caractérisé en ce qu'**un dispositif d'entrée et/ou de sortie (12), notamment un écran de thérapie, est fixé de manière mobile au boîtier de bras pivotant (6).

9. Système de thérapie ophtalmique au laser (100) selon l'une des revendications 1 à 8, le dispositif d'examen (15) étant un microscope chirurgical.

10. Système de thérapie ophtalmique au laser (100) selon la revendication 8 ou 9, caractérisé en que le dispositif d'entrée et/ou de sortie (12) peut tourner autour d'un axe (17, 18) parallèle au premier axe (4) du boîtier de bras pivotant (6) et/ou le dispositif d'examen (15) peut tourner autour d'un axe (17, 18) parallèle au deuxième axe (16) du bras pivotant d'examen (14), et est couplé par le biais d'un arrangement d'accouplement (19) au mouvement du boîtier de bras pivotant (6) ou du bras pivotant d'examen (14).

11. Système de thérapie ophtalmique au laser (100) selon l'une des revendications 1 à 10, **caractérisé en ce que** la première et/ou la deuxième optique de thérapie au laser contient un système de balayage comprenant au moins deux scanners, et le premier axe (4) du bras pivotant de laser (3) est disposé dans l'axe du faisceau entre les scanners.

12. Système de thérapie ophtalmique au laser (100) selon l'une des revendications 1 à 11, **caractérisé par** un arrangement de régulation de position destiné à réguler la position entre le bras pivotant de laser (3) et/ou le bras pivotant d'examen (14) en position de travail et l'œil d'un patient, lequel est conçu pour asservir la position de la tête d'appareil (1) à la position de l'œil d'un patient.
